# EUROPEAN PATENT SPECIFICATION

(11) **EP 2 995 548 B1**
(45) Date of publication and mention of the grant of the patent: **16.01.2019**
(21) Application number: 15189530.7
(22) Date of filing: 20.02.2014
(51) Int. Cl.: B63C 11/24, B63C 11/26, A62B 7/10, A62B 9/06, A62B 25/00

(54) **REBREATHER SYSTEM AND COMPONENTS**
ATEMSYSTEM UND KOMPONENTEN
SYSTÈME ET CONSTITUANTS DE RÉINHALATION

(30) Priority: 22.02.2013 GB 201303182
(43) Date of publication of application: 16.03.2016
(62) Divisional of application: 14705533.9
(73) Proprietor: Johnson Outdoors Inc., Racine, WI 53403 (US)
(72) Inventor: Sieber, Arne, 5700 Zell am See (AT)
(74) Representative: Cohausz & Florack

(56) References cited:
- EP-A1- 1 953 081
- US-A1- 2003 115 010
- US-A1- 2012 065 486
- US-B1- 6 447 115

## Description

The invention relates to a counterlung arrangement for a rebreather, a mouthpiece for a rebreather, a transport device for a rebreather, a display arrangement for a diving apparatus such as a rebreather, and a rebreather for supplying a user with a breathing gas.

A breathing apparatus is necessary whenever a person is exposed to a life threatening or non life sustaining environment. A major part of a life supporting system is the breathing apparatus necessary to supply breathing gas when an individual is exposed, for example, to water (diving) or to hazardous gases (firefighting). The gas supply system is often built around the tanks with compressed gas. A regulator system reduces the pressure from the tanks to ambient pressure. An individual is breathing normally via an "on demand" valve, which provides fresh breathing gas from the tank, whenever the person inhales. Such systems are called open circuit breathing systems (short OC), as the exhaled gas is vented through another valve into the environment.

An open circuit breathing system used in SCUBA diving consists therefore of the following parts:
- Tank (200 - 300 bar compressed breathing gas supply)
- Pressure regulator mounted on the tank, which reduces the tank pressure to a pressure of typically 8-10 bar over ambient pressure which is equal to the surface atmospheric pressure plus the hydrostatic pressure. This pressure regulator is typically referred in diving as first stage.
- An "on demand" valve, which provides breathing gas to the diver. This device is usually referred as "second stage". A downstream OC valve is a common type of such "on demand" valves.
- The first and the second stages are connected to each other via a breathing hose.

The amount of time within which the system can provide breathing gas depends on the amount of gas stored in the tanks, the gas volume used in every breath and the respiratory rate. Typical breathing volumes (RMV, respiratory minute volume) are 5-10 l/min RMV at rest. During light activities, the RMV may be around 12-15l /min. During moderate exercise the RMV can rise up to 40-60 l/min. As only a small portion of the breathing gas is metabolized - in fact only 0.3-0.8 l/min O₂ at rest and up to 3.5 l/min O₂ under heavy work are taken up by the human body, the gas efficiency of open circuit systems is quite low, only about 3 %. Especially when it comes to diving, where the ambient pressure is increased by 1 bar each 10 m depth due to the additional hydrostatic pressure, the efficiency even decreases and may reach values of about 0.6 % at 40 m depth. To enable deep and long dives, a sufficient gas supply is required, including in many cases several tanks resulting in bulky and cumbersome equipment.

Closed-circuit rebreathers have many advantages in comparison to open circuit systems. In an oxygen rebreather a person exhales into a bag - the so called counterlung. A scrubber removes carbon dioxide (CO₂) and fresh gas is added to replace metabolized oxygen (O₂). This recycled gas is then inhaled by the diver again. In the case of a O₂ rebreather, the circuit contains mainly O₂ and traces of N₂. Thus the partial pressure of O₂ (pO₂) inside the circuit is dependent on the ambient pressure (Dalton's Law). Such a system has the advantage of increasing the gas efficiency up to 100%. O₂ rebreathers can be designed as purely mechanical systems, and are robust and reliable. Many rebreathers require mixture of oxygen and other gases for respiration. For example in the case of firefighting, one would tend to avoid breathing systems containing pure O₂ because of the increased risk of combustion. In diving applications the use of pure oxygen is only advisable to a maximum depth of 6m, as O₂ becomes toxic at partial pressures greater than 1.4 - 1.6 bar. In diving, a diluent gas is used to lower the partial pressure of O₂ (short pO₂). This diluent gas is typically air or so called TRIMIX, containing He, O₂ and N₂. Closed circuit re-breather systems (short CCR) that use a gas mixture cannot be purely mechanical since in that case pO₂ monitoring and regulation is required. Wet - electrochemical pO₂ sensors are used to measure pO₂. A manual or automatic control loop is used to keep the pO₂ at constant level by replacing metabolized O₂ with fresh O₂ from a supply tank.

OC regulator design and CCR model descriptions can be found in many textbooks for professional, technical as well as recreational diving, for example the US Navy Diving manual or the NOAA diving manual.

Typically, a rebreather consists among others of the following components:
- A mouthpiece with direction valves
- One inhale and one exhale hose which lead to the counterlungs
- An exhale counterlung and an inhale counterlung
- One scrubber canister in which a CO₂ filter is housed
- Hose connection between the scrubber canister and the counterlungs
- Manual diluent valve or automatic diluent valve (ADV)
- Loop Overpressure valve
- Sensor and electronic compartment on top of the scrubber canister
- Handset to display dive relevant data
- Head-up display (HUD)
- Harness
- Buoyancy compensator

When the diver descends, the loop volume is decreased due to the increasing ambient pressure. This has to be compensated with an injection of diluent, as the breathing volume remains the same independent on the depth of the diver. For this reason, rebreathers are equipped with a manual diluent valve, which allows injection of diluents into the loop. Alternatively some rebreathers are equipped with an automatic diluent valve (ADV), which works similar to a second stage OC breathing regulator - it automatically injects diluent in the loop as soon the loop is empty and there is a negative pressure. Typically the pressure threshold at which the diluents injection is started is set to -20 to -40 mbar. This threshold is called "cracking pressure". In contrast to that, the cracking pressure of an OC second stage is typically set to -1 to -3 mbar.

The loop pressure inside a rebreather is dependent on the divers angular position (pitch and roll) and on the position of the counterlungs.

The ADV in a rebreather is set to such a high cracking pressure in order to avoid unwanted gas injections in some positions.

When the diver ascends, the volume of the gas in the loop increases due to the decreasing ambient pressure. An overpressure valve is located in the loop in order to release excessive gas. Such a valve is typically set to a value of +10 to +40 mbar.

A CCR is usually also equipped with one or more pO₂ sensors to monitor the pO₂ inside the loop. The pO₂ values are typically shown on a small display. In most rebreathers an additional head-up display is used, which is located on the rebreather mouthpiece in a position where it is always in the field of view of the diver. These are very basic displays consisting of one or more LEDs.

In the case of a malfunction of the rebreather, the pO₂ inside the loop can quickly reach values outside life sustaining thresholds. In such cases the HUD warns the diver which then should "bail out" as first response. Under "bail out" a rebreather diver understands to continue breathing from an OC second stage regulator.

The response to an alarm of a HUD indicating a life threatening condition should be executed fast. Changing from the rebreather mouthpiece to an OC second stage requires multiple tasks - first the diver has to grab the second stage, close the rebreather mouthpiece and then change to the OC second stage. It is important to close the rebreather mouthpiece to avoid flooding of the rebreather loop. The task, changing from a rebreather mouthpiece to an OC regulator can be very demanding, especially if the rebreather diver is already impaired or in a condition of dizziness, which can occur when the diver is breathing a gas outside life sustaining limits (like for example high CO₂ concentration, too high nitrogen partial pressure, oxygen intoxication, etc).

In order to facilitate changing from closed circuit breathing (CCR) to open circuit breathing mode (OC), rebreather mouthpieces were developed which feature two different modes - one CCR mode, in which the diver breathes from the closed circuit rebreather, and an OC mode. To realize an OC function, the parts of a second stage regulator are integrated into the mouthpiece (diaphragm, diaphragm actuated valve, exhaust valve). Typically the diver switches between the two modi by rotating a barrel inside the mouthpiece.

US 5,127,398 discloses a rebreather mouthpiece which can be switched between open circuit and closed circuit mode.

WO 2007/126317 discloses the first attempt of a rebreather mouthpiece with OC and CCR mode but additional automatic diluents valve (ADV). However, there the cracking pressure of the ADV is not increased - instead the gas path is restricted. This does however not give the same result as an increased cracking pressure, thus the ADV will free flow in some rebreather designs (especially in designs with back mounted counterlungs).

GB 2,340,760 discloses a rebreather mouthpiece with OC and CCR mode but also with an ADV. However, here the cracking pressure of the ADV is not increased.

GB 2,462,672 discloses a rebreather mouthpiece which can be switched between OC and CCR mode and has additionally an integrated ADV function. The cracking pressure is automatically adjusted to a higher pressure in CCR mode by changing the membrane active area, the leverage ratio of the diaphragm to valve seat pressures or the valve seat spring tension.

EP 2,207,715 discloses the design of a rebreather mouthpiece with integrated OC and CCR mode and integrated ADV. Here, the cracking pressure for the ADV is increased in comparison to the OC cracking pressure. The function therefore is realized by moving the pilot valve relative to the diaphragm. In CCR mode the pilot valve is moved away from the diaphragm, thus a decrease of the active diaphragm area is achieved which results in a higher cracking pressure.

Head-up displays currently used in rebreathers are typically LED based. An LED however has limited information content - therefore different colors and blinking sequences are used.

EP 2,207,715 discloses a head-up display which also senses the mode of the mouthpiece (OC or CC mode).

Divers are possibly already impaired or in a condition of dizziness when a HUD indicates and alarm. In such a condition, the diver might misinterpret LED based HUDs, especially when the information content was increased with blinking sequences, etc. which require training for correct interpretation. An alternative to LED based HUDs is disclosed in GB 2,427,364. Here, synthetic speech clips are used to warn the diver.

Austrian Utility Patent GM267/2008 discloses a graphical display with an optical system mounted directly onto the mouthpiece of a rebreather. With a carefully positioned device, good readability of standard dive data is achieved. However, mouthpiece movements resulted in optical misalignments where the display (partly) moved out of sight.

Gallagher and Belcher (Gallagher, D.G. (1999). Development Of Miniature, Head-Mounted, Virtual Image Displays For Navy Divers. OCEANS '99 MTS/IEEE, Riding the Crest into the 21st Century, Vol. 3, pp. 1098-1104) developed Head Mounted Displays for counter mine diving missions. The designs are large, expensive in production and consume significant power.

WO/2010/076177 A1 discloses a head-up display for conventional diving masks. The device consists of a 1" white OLED screen mounted on an acrylic light path and is directly glued to the visor of a diving mask. Inside the mask a prisma shaped convex lens is mounted. In that way, a virtual image distance of 1m is achieved. The monocular HMD only occludes a small peripheral area of the divers field of view thus causes relatively little optical interference. The unique design, which enables attachment to almost any diving mask on the market furthermore allows outstanding pressure resistance and at the same time cost effective manufacturing. The device is designed as a secondary display to a primary wrist worn diving computer. A cable connection between the handset and the head mounted display is required. Drawbacks of that device are twofold. On the one hand, users find the cable connection between the head mounted display and the wrist worn diving computer disturbing and/or annoying. The second problem is that once the device is glued to the mask it cannot be adjusted anymore. Especially as the device offers high magnification of the little OLED screen, this is a big problem, as small misalignments of the display in respect to the user's eye will lead to an impaired reception of the virtual image, where some corners or parts of the virtual image are cropped. More information can be found in: Koss B, Sieber A, Head Mounted Display for Diving Computer Platform. IEEE Journal of Display Technology, Vol 7, Issue 4, pp 193-199,2011

The Aeris CompuMask (R) HUD and the Oceanic DataMask (R) HUD are recreational diving computers which are fully integrated into a diving mask. The displays offer excellent readability. However the system is designed as a closed system and therefore cannot be adapted to rebreathers.

GB 2427366 describes an infrared link for fault tolerant rebreather electronics.

In WO 2012/035021 A1 an optical tank pressure data transmission between tank pressure sensor and transmitter is mentioned.

US 6,447,115 B1 discloses a dive mask display system for use in monitoring dive information. The system comprises a dive mask having at least one lens defining a field of view and a frame supporting said at least one lens. A plurality of components integrated in said frame, such as a radio frequency (RF) antenna, a display, a controller and an input device. The display shall be positioned outside of the field of view. The RF signals are indicative of dive information and are received by the RF antenna.

EP 1 953 081 A1 discloses a head-up display unit comprising a head-up display and further a receiver. The head up display makes use of LED or LCD technology. The data receiver and transmitter are induction transmitters for communicating wirelessly.

US 2012/065486 A1 discloses a system for monitoring biometric data of a diver. The biometric data (e.g. blood oxygen saturation level) may be detected by optical transmitters/detectors. For data communication, it acoustic or magnetic communication is suggested.

US 2003/0115010 A1 relates to an underwater diving mask with a visual display. Communication to the display is realized by a cable between the display and the computer system.

State of the art in closed circuit rebreather are relatively bulky systems, which are heavy, cumbersome and due to this difficult to transport.

Open circuit diving apparatus are simple and easy to prepare for diving. In contrast to that, preparation of a rebreather includes next to assembly of all the parts also several checks to see for instance if all sealings work correctly, if the electronic gives correct readings and if the tanks are sufficiently filled. Closed circuit rebreather are rather complex diving systems, mainly due to the large amount of parts.

After a dive, a rebreather has to be disassembled, cleaned and disinfected and dried. Due to the amount of parts post dive maintenance takes rather long from half up to one hour.

Currently only few rebreathers are sold in comparison to open circuit diving equipment. One major reason for this is that rebreathers are far more expensive. One has to afford typically more than five times the price of an open circuit equipment. One reason for this is that today's rebreathers consist of many complex parts, and therefore the fabrication of is costly, which results in a high end user price.

Recreational divers usually do not dive in their home country. They prefer travelling by plane to distant destinations. If a recreational diver owns his own rebreather, he most likely would like to bring it with him. However, current systems are heavy and large, thus the only way to transport a rebreather is typically in checked luggage. As most airlines have weight limits for checked luggage, a rebreather diver often has to pay additionally for sports equipment or additional weight. Many times divers do not like to check luggage with costly equipment - like breathing regulators, torches or cameras, as often checked luggage gets lost.

Current drawbacks of closed circuit rebreathers are therefore:
- Rebreathers are heavy and cumbersome and difficult to transport on airplanes. A rebreather typically cannot be brought in the cabin luggage.
- Rebreathers consist of many complex parts, therefore preparation and post dive maintenance takes far more time than for open circuit diving systems
- Rebreathers consist of many complex parts, therefore the production costs are high which results in a high end user price.
- Long hoses, many hose connections and couplings increase the breathing resistance and work of breathing.

Hence, it is still difficult to provide a rebreather system which does not suffer from the above shortcomings.

It is an object of the invention to provide diving apparatus/rebreather components as well as a rebreather which overcome at least a part of the above described drawbacks.

In order to achieve the object defined above, diving apparatus/rebreather components as well as a rebreather according to the independent claims are provided.

According to an exemplary embodiment of a first aspect of the invention, a mouthpiece for a rebreather being operable in a closed circuit mode or in an open circuit mode is provided, the mouthpiece comprising an inhale direction valve (which may be connected to a counterlung, particularly to an inhale counterlung section of the counterlung), an exhale direction valve (which may be connected to the counterlung, particularly to an exhale counterlung section of the counterlung), an automatic diluent valve (which may be connected to a breathing gas supply bottle), an overpressure valve (which may operate between an interior of the mouthpiece and an environment such as water in case of diving) and a switching barrel switchable by a user between a closed circuit switch state for operating the mouthpiece in the closed circuit mode and an open circuit switch state for operating the mouthpiece in the open circuit mode so that in the closed circuit switch state, a user is enabled to inhale breathing gas via the inhale direction valve (particularly connected to the counterlung) and to exhale breathing gas via the exhale direction valve (particularly connected to the counterlung) wherein the overpressure valve opens beyond a first threshold pressure (particularly opens only when an overpressure in an interior of the mouthpiece exceeds the first threshold pressure) and wherein the automatic diluent valve provides a diluent gas beyond a second threshold pressure - also denoted as a cracking pressure - (particularly opens only when the absolute value of a negative pressure in an interior of the mouthpiece exceeds the second threshold pressure), and in the open circuit switch state, a user is enabled to inhale breathing gas via the automatic diluent valve (and hence via a connected gas bottle) and to exhale breathing gas via the overpressure valve, wherein the overpressure valve is deactivated or the absolute value of the first threshold pressure is reduced (particularly the overpressure valve is converted into a configuration in which the first threshold pressure has a smaller value, i.e. opens already at a smaller overpressure value) in the open circuit switch state (which deactivation and hence conversion to temporarily function as an outlet valve may be the direct result of the switching actuation of the switching barrel) and wherein the automatic diluent valve provides a diluent gas beyond a third threshold pressure - also denoted as a further cracking pressure - (particularly opens only when the absolute value of a negative pressure in an interior of the mouthpiece exceeds the third threshold pressure) having a smaller absolute value than the second threshold pressure (particularly, the threshold or barrier against the supply of fresh diluent gas or breathing gas may be higher in the closed circuit mode than in the open circuit mode).

According to such an embodiment, a mouthpiece for a breathing apparatus is provided, which includes an open circuit and a closed circuit mode, an automatic diluent valve and a loop overpressure valve. The automatic diluent valve is configured to allow the addition of the diluent gas at two different threshold pressure values in the closed circuit mode and in the open circuit mode. In the open circuit mode, fresh diluent gas is delivered already when a user generates a very small negative pressure in the mouthpiece by inhaling (in OC mode, the loop is isolated from the user). In the closed circuit mode, fresh diluent gas is delivered only when the user generates a higher negative pressure in the loop by inhaling. Moreover, such an embodiment uses an overpressure valve with this function only in the closed circuit mode, whereas in the open circuit mode the overpressure valve is actuated so as to be open, therefore now functioning as an outlet valve or exhale valve.

Alternatively, the overpressure valve may be designed with two different threshold pressure values, one high pressure for CCR mode and one lower threshold pressure value for OC mode, in order to avoid a separate exhaust valve.

According to an exemplary embodiment of a second aspect of the invention, a counterlung arrangement for a rebreather is provided, wherein the counterlung arrangement comprises an integrally formed counterlung (which may be made of a flexible material which can be stretchable or non-stretchable) formed as a single piece (such as a single bag with, when no filter is inserted into the interior of the single bag, a single interior compartment) and comprising an inhale counterlung section to be connected to an inhale side of a mouthpiece, an exhale counterlung section to be connected to an exhale side of the mouthpiece, and a filter accommodation section arranged between he inhale counterlung section and the exhale counterlung section and being configured for accommodating a breathing gas filter cartridge, wherein the counterlung arrangement further comprises the breathing gas filter cartridge configured for filtering breathing gas flowing between the exhale counterlung section and the inhale counterlung section when being accommodated in the filter accommodation section, wherein the counterlung and the breathing gas filter cartridge are configured to match to one another so that the breathing gas filter cartridge is sealingly arrangable (for instance clampable or so that the breathing gas filter cartridge seals even without clamping) between the exhale counterlung section and the inhale counterlung section in an interior volume of the counterlung (particularly to thereby establish breathing gas cleaning of breathing gas propagating from the exhale counterlung section via the filter cartridge to the inhale counterlung section, while the filter is sealed within the counterlung by a circumferential sealing force exerted (directly or indirectly by an external clamp) from the counterlung material to the filter, thereby dividing the interior volume of the counterlung into the exhale counterlung section and the separate inhale counterlung section only by inserting the filter in the counterlung).

According to such an embodiment, a rebreather is provided with a single piece counterlung, which has an inhale side and an exhale side and in which a breathing gas filter cartridge such as a CO₂ filter is inserted and is held between the two counterlung sections solely by a sealing effect of a sandwiched seal or a clamping force exerted onto the breathing gas filter cartridge by the surrounding counterlung itself. In the closed circuit mode, a closed loop is formed by the mouthpiece being (via an inhale direction valve and via an exhale direction valve, respectively) in gas communication with the two counterlung sections which are in turn separated by the filter cartridge. Hence, a user inhales breathing gas from the inhale counterlung section and exhales the breathing gas via the exhale counterlung section. By this breathing action, carbon dioxide is enriched in the breathing gas and is at least partly removed while passing the carbon dioxide filter cartridge. The configuration of the counterlung and the corresponding breathing gas filter cartridge allows very simple handling of the rebreather by a user.

According to an exemplary embodiment of a third aspect of the invention, a transport device for a rebreather is provided, the transport device comprising a harness configured to be wearable by a user by fastening the harness at an upper part of the body of the user, rebreather components mounted on the harness so as to be arranged at the back of the user when wearing the harness, and a rebreather component cover configured for covering at least a part of the rebreather components, wherein the harness and the rebreather component cover are provided with mutually matching fastening elements being configured so that the rebreather component cover is selectively fastenable to the harness to thereby cover at least a part of the rebreather components for transportation (particularly in the cabin of a plane) or detachable from the harness to thereby expose the rebreather components for using the rebreather (for instance for a task such as diving or firefighting).

According to such an embodiment, a rebreather with a harness is provided, which can be easily transformed into a backpack by merely fastening the rebreather component cover at the harness.

According to an exemplary embodiment of a fourth aspect of the invention, a display arrangement for a diving apparatus, particularly for a rebreather, is provided, the display arrangement comprising a first communication partner device having a first mounting element to be mounted at a first position of the diving apparatus, particularly on a mouthpiece or a breathing hose of the diving apparatus, a second communication partner device configured as a head-up display (particularly an electronic head-up display) for visually displaying data to a user and having a second mounting element to be mounted at a second position of the diving apparatus so that the head-up display is in the field of view of the user, and a wireless communication link (such as a wire-free communication connection) configured for transmitting the data via a light signal indicative of the data from the first communication partner device to the second communication partner device.

According to such an embodiment, one diving apparatus (such as rebreather) communication partner device (which may be but does not have to be a head-up display) may be located on the mouthpiece, wherein light signals such as light pulses (using light of a suitable wavelength in the visible range, the infrared range, or any other suitable range) are used in order to transmit wireless rebreather or other diving-relevant data to a head-up display (which also functions as a further communication partner device) which is for instance located on the diver's mask. Such a wireless communication in the infrared or visible domain is not disturbing and very convenient for a user of the diving apparatus and is promoted by the close spatial relationship between the two communication partner devices (particularly two head-up displays) which may be both in the field of view of the user and being therefore also enabled for mutual communication.

According to an exemplary embodiment of a fifth aspect of the invention, a rebreather for supplying a user with a breathing gas is provided, wherein the rebreather comprises at least one of a mouthpiece having the above mentioned features, a counterlung arrangement having the above mentioned features, a transport device having the above mentioned features, and a display arrangement having the above mentioned features.

According to such an embodiment, it is possible to provide a rebreather with a multifunctional mouthpiece with a closed circuit mode, an open circuit mode, an automatic diluent valve with a high cracking pressure particularly in the closed circuit mode, and an overpressure valve particularly deactivatable/reducible in terms of opening threshold value in the open circuit mode, wherein by simply turning a barrel the modes can be selected and the automatic diluent valve and the overpressure valve get actuated synchronously. Further said rebreather may have a single piece counterlung, in which a filter cartridge such as a CO₂ filter cartridge is inserted through an opening and placed in between inhale and exhale side of the counterlung, to achieve a double counterlung system. The rebreather can furthermore be transformed into a backpack, to achieve good protection and enable easy transportation of the device even in the cabin of an airplane or the like. The rebreather may be equipped with a primary communication partner device (such as a for instance LED based head-up display) and a secondary communication partner device having a head-up display, wherein the primary communication partner device may be located on the mouthpiece and the secondary, for instance OLED or LCD based head-up display may be located on the frame of the diver's mask, and the primary communication partner device may transmit dive relevant data via an optical link, preferable infrared of visible light, to the secondary head-up display.

A rebreather having features in accordance with one or more of the above described aspects and which may particularly be used for recreational purposes may have the following advantages:
- A small amounts of parts is sufficient, so that assembly and maintenance is simplified.
- The rebreather can be manufactured with low weight.
- The rebreather can be manufactured with compact size so that it can be transported in an airplane in the cabin luggage.
- Even more complex parts are designed in a cost efficient way, so that injection molded fabrication can be done with simple and rather cheap molds. This refers especially to the mouthpiece.
- The mouthpiece is switchable between OC and CCR mode, offers an ADV function and reduces the amount of parts of the rebreather, and includes also a loop overpressure valve.
- Parts may be designed in a way, so that the breathing resistance/work of breathing is low, thus the amount of hoses and hose connections is limited to a small number.
- The rebreather can be equipped with a very simple LED based primary head-up display, which gives a simple to understand warning in the case of a problem.
- The rebreather can also be equipped with a secondary HUD with a little screen without a cable connection and with the ability to adjust the device easily in respect to the divers eyes.

In the following, further exemplary embodiments of the first aspect will be explained which also hold for the second, the third, the fourth and the fifth aspect.

In an embodiment, the mouthpiece comprises a two part housing accommodating the switching barrel being switchable between the closed circuit switch state and the closed circuit mode by turning the switching barrel within the two part housing. In an embodiment, each of the two parts of the housing is manufactured by injection molding and the two parts are bonded together to thereby form the two part housing. The mouthpiece housing may thus be provided with a split line through a middle portion thereof. Such housing parts can be fabricated in a simple mold.

In an embodiment, the automatic diluent valve is configured to provide a diluent gas (which may form the or may form part of the breathing gas) in the closed circuit switch state in the presence of a negative pressure in a range from about -20 mbar to about -50 mbar as the second threshold pressure. Thus, the mouthpiece may have a cracking pressure of the automatic diluent valve which is preferable as high as -20 to -50 mbar with a lever which excerpts a force on a diaphragm of the automatic diluent valve. Therefore, unwanted addition of diluent gas resulting from very minor negative pressure in the loop is prevented in the closed circuit mode by adjusting the cracking pressure to a relatively high value.

In an embodiment, the automatic diluent valve is configured to provide a diluent gas (which may form the or may form part of the breathing gas) in the open circuit switch state in the presence of a negative pressure in a range from about -0,1 mbar to about -5 mbar as the third threshold pressure. The mouthpiece may hence have a cracking pressure of the open circuit mode which is preferable between -0.1 and - 5 mbar. Therefore, the user does not have to exert a high breathing work in the open circuit mode so that already a relatively low negative pressure in a void volume of the mouthpiece is sufficient to trigger the addition of diluent gas.

In an embodiment, the automatic diluent valve comprises a threshold adjustment mechanism configured for adjusting the second threshold pressure. Therefore, the second threshold pressure can be adjusted to a desired or user defined value for instance by selecting a different spring length and/or different spring constant. This increases the flexibility of a user to adjust the rebreather to user preferences.

In an embodiment, the threshold adjustment mechanism comprises a spring-loaded lever (or any other biasing element) configured for applying a counterforce (as compared to a breathing force) to a diaphragm in the closed circuit switch state, wherein the switching barrel is configured so that upon switching from the closed circuit switch state into the open circuit switch state, the counterforce is released from the diaphragm, particularly by pulling the spring-loaded lever (which has exerted the counterforce) away from the diaphragm (for instance by a filament being tensioned by the switching actuation). The mouthpiece may be provided with a barrel, which can be set in either open circuit or closed circuit position, whereas in the closed circuit position an additional force is excerpted with a spring loaded lever of the threshold adjustment mechanism on the diaphragm in order to increase the cracking pressure to preferable -20 to -50 mbar. In the open circuit position, this lever may be pulled inside the mouthpiece to unload the diaphragm and achieve a cracking pressure of preferable 0.1 to 5 mbar.

In an embodiment, the overpressure valve comprises a plate covering a gas passage (such as a small tubular element tube with a sealing thereon) and comprises a biasing element, particularly a spring, biasing the plate to cover the gas passage in the closed circuit switch state so as to open only beyond the first threshold pressure to thereby execute its overpressure protection function. In an embodiment, the biasing element is connected between the plate and the switching barrel, particularly a lever of the switching barrel, so that, upon switching the switching barrel into the open circuit switch state, the biasing force is released from the plate. Therefore, the barrel, which can be set in either open circuit or closed circuit position, pulls in the closed circuit position a plate against a sealed seat to achieve an overpressure valve, which opens at an adjustable value. In the closed circuit position said plate is released, the overpressure valve opens and this opening serves together with a one way valve as exhaust valve. In other words, this opening of the overpressure valve corresponds to its deactivation in the open circuit mode.

In an embodiment, the overpressure valve is configured to open in the closed circuit switch state in the presence of an overpressure in a range from about 10 mbar to about 50 mbar as the first threshold pressure. More particularly, the mouthpiece may be foreseen with an overpressure valve which opens at an overpressure of preferable 15 mbar to 45 mbar.

In an embodiment, the mouthpiece comprises a purge button, particularly forming part of the automatic diluent valve, configured for actuating the automatic diluent valve to thereby activate a manual activation mode of the automatic diluent valve. The provision of such a purge button extends the flexibility of the user to configure the mouthpiece in accordance with user preferences.

In the following, further exemplary embodiments of the second aspect will be explained which also hold for the first, the third, the fourth and the fifth aspect.

In an embodiment, the counterlung comprises or exclusively consists of an elastically stretchable material, particularly neoprene rubber, so that the elasticity of the counterlung provides (alone or in combination with a further clamping mechanism) for the clamping force for sealingly clamping the breathing gas filter cartridge between the exhale counterlung section and the inhale counterlung section. The counterlung can hence be designed from flexible material, preferable neoprene rubber. The elasticity of this material itself provides the biasing force which engages the filter cartridge when being inserted in the counterlung, particularly into the filter accommodation section thereof.

In the absence of the breathing gas filter cartridge, a gap between the exhale counterlung section and the inhale counterlung section at the position of the filter accommodation section may be smaller than an extension of the breathing gas filter cartridge separating the exhale counterlung section and the inhale counterlung section from one another when the breathing gas filter cartridge is accommodated in the filter accommodation section. Thus, when inserting the filter cartridge between the two counterlung sections, a user first has to slightly increase the volume between the counterlung sections by the insertion force so that the filter cartridge may be placed in the widened filter accommodation section so that the counterlung self-sufficiently engages the filter cartridge to apply the fastening clamping force. Thus, the filter accommodation section in between the inhale side and the exhale side of the counterlung has a smaller circumference than the one of the scrubber or filter cartridge. The elasticity of the material may be used to seal the inhale and exhale side to avoid gas bypassing the filter cartridge.

In an embodiment, the counterlung is made of a non-stretchable material such as HF (high-frequency) weldable polyurethane of PVC foil or polyurethane of polyvinylchloride coated fabric. This guarantees a simple manufacturability of the counterlung. Upon inserting the filter into the filter accommodation section, there will remain a gap in between the filter and the filter accommodation section which is advantageous for a convenient insertion of the filter into the counterlung. For a proper sealing it is advantageous to close the gap with an external clamp.

In an embodiment, the counterlung arrangement further comprises a clamp, particularly an elastically stretchable or non-stretchable string or a mechanically rigid clamp, to be mounted or wound around the filter accommodation section and the breathing gas filter cartridge to thereby provide a clamping force for sealingly clamping the breathing gas filter cartridge in the filter accommodation section between the exhale counterlung section and the inhale counterlung section. A sealing can hence be established with a flexible string (such as an elastic band, for instance of rubber) wound around the filter cartridge outside the counterlung. The flexible string may however also be made of a non-stretchable but bendable material. It is also possible to use a mechanical clamp such as a pipe clamp.

In an embodiment, the counterlung arrangement further comprises a clamp, which is mounted on the filter accommodation section, in order to achieve a proper sealing of the counterlung material to the gas filter cartridge.

In an embodiment, the counterlung comprises a closable access opening configured for providing an access for the insertion of the breathing gas filter cartridge into an interior volume of the counterlung, and a (for instance locally narrowed) section of the interior volume configured for clampingly holding the breathing gas filter cartridge after its complete insertion into the interior volume via the access opening. The access opening may be opened for insertion of the breathing gas filter cartridge. The access opening may be sealingly closed when the breathing gas filter cartridge is inserted into an interior volume of the counterlung to clean breathing gas streaming from the exhale counterlung section via the filter towards the inhale counterlung section. The narrow portion or bottleneck may be located between the inhale counterlung section and the exhale counterlung section so as to provide for an interface-less gas connection between the counterlung sections and the filter.

In an embodiment, the access opening has larger dimensions than the breathing gas filter cartridge to simplify its insertion into the interior volume of the counterlung. Thus, when the filter cartridge is inserted into the interior of the counterlung, there may still remain a gap when the filter passes the access opening.

In an embodiment, a transition between the inhale counterlung section and the accommodated breathing gas filter cartridge is free of any connection member and/or a transition between the exhale counterlung section and the accommodated breathing gas filter cartridge is free of any connection member. Therefore, there may be a direct gas flow connection between the filter material and the counterlung sections without any hoses or other connection members there between.

In an embodiment, the breathing gas filter cartridge is a carbon dioxide filter cartridge. Such a carbon dioxide filter cartridge is configured for filtering carbon dioxide out of the breathing gas so as to increase the time over which a given amount of breathing gas can supply a user before exchange of a gas bottle or the like.

In an embodiment, the counterlung and the breathing gas filter cartridge may be configured to match to one another so that the breathing gas filter cartridge seals between the exhale counterlung section and the inhale counterlung section in an interior volume of the counterlung. In particular, sealing of the breathing gas filter cartridge against a corresponding counterlung wall in the filter accommodation section may be advantageously achieved with at least one sealing, preferable at least one O-ring and/or at least one lip sealing. In an embodiment, a sealing may be mounted between the inhale section and the exhale section of the counterlung, which seals the CO₂ filter cartridge, so that no gas flowing from exhale section to inhale section of the counterlung can bypass the CO₂ filter. The sealing can be achieved with for instance a ring mounted inside the counterlung. When the CO₂ filter cartridge is inserted, O-rings, lip sealings or other sealings can be used to seal the CO₂ filter against the counterlung.

In the following, further exemplary embodiments of the third aspect will be explained which also hold for the first, the second, the fourth and the fifth aspect.

In an embodiment, the harness and the rebreather component cover are configured to forming together a backpack when the rebreather component cover is fastened to the harness. A backpack may comprise a cloth sack (forming part of the harness) carried on a user's back and secured with two straps (also forming part of the harness) that go over the shoulders. Such a backpack can be carried by a user on her of his back by means of usually two straps connecting a bag section on the back with the breast of the user. The bag contains the rebreather components, i.e. the components required for fulfilling a rebreather functionality.

In an embodiment, the matching fastening elements form a zip fastener, particularly a circumferentially closed zip fastener. Hence, a rebreather can be provided in which the backpack outer shell is mounted with a circumferencing zip and where the counter part of the circumferencing zip is located on the rebreather. A zip fastener may denote a device for binding the edges of an opening of fabric or other flexible or rigid material. A zipper or zip comprises two rows of protruding teeth which may be made to interdigitate, linking the rows, carrying from tens to hundreds of specially shaped metal or plastic teeth. These teeth can be either individual or shaped from a continuous coil. A slider, operable by hand, moves along the rows of teeth. Inside the slider is a for instance basically Y-shaped channel that meshes together or separates the opposing rows of teeth, depending on the direction of the slider's movement.

In an embodiment, the matching fastening elements form a Velcro fastener which will also be denoted as hook-and-loop fastener. Thus, a rebreather may be provided in which the backpack outer shell is mounted with Velcro. Particularly, such a Velcro fastener may have hook fastening elements on one of the rebreather component cover and the harness and may have cooperating loop fastening elements on the other one of the rebreather component cover and the harness. Hook-and-loop fasteners may be formed of two components. Typically, two fabric strips (or, alternatively, round dots or squares) are attached (for instance sewn, adhered, etc.) to the opposing surfaces to be fastened. The first component features tiny hooks. The second features loops. When the two components are pressed together, the hooks catch in the loops and the two pieces fasten or bind temporarily.

In an embodiment, the rebreather component cover is configured as a protective shell, particularly a protective stiff shell. In such an embodiment, the cover may protect the rebreather components also against beats or other mechanical loads. Soft cushions or the like may be provided on the stiff shell as well so as to dampen mechanical loads. In an alternative embodiment, the rebreather component cover is configured as a fabric, particularly a bendable fabric.

In an embodiment, the rebreather component cover comprises at least one pocket, particularly arranged at an internal surface of the rebreather component cover facing the rebreather components or at an external surface of the rebreather component cover being exposed to an environment. Tickets, passports, purses, etc., but also detached rebreather components can be stored in such pockets.

In an embodiment, the rebreather components comprise a counterlung, a mouthpiece, a breathing gas filter cartridge, a breathing gas tank, a diluent gas tank, a buoyancy compensator, one or more head-up displays, one or more breathing hoses and/or a mask (which may have a visor and a frame). However, it is possible that the cover only covers one or any desired subset of these and/or other rebreather components. For instance, the transport arrangement may be configured so that the mouthpiece and optional hoses may first be removed from the rebreather (and stored in pockets or the like) before connecting the cover and the harness.

In the following, further exemplary embodiments of the fourth aspect will be explained which also hold for the first, the second, the third, and the fifth aspect.

In an embodiment, it is possible to use a head up display on the mask with the infrared link but without a primary head up display (so for example in a situation, where only one infrared diode is mounted on the regulator).

In an embodiment, a communication link arrangement for a display is provided, with a display being preferable a head up or near eye display in close vicinity to the eye for visually displaying data and the communication link being a wireless communication link where data are transmitted via light to said display.

In an embodiment, the data is transmitted from a mouthpiece to the display. The mouthpiece or alternatively breathing hose are appropriate positions for mounting the communication partner device.

In an embodiment, the first communication partner device is configured as a further head-up display for visually displaying further data to the user, wherein the first mounting element is to be mounted at the first position of the diving apparatus so that the further head-up display is in the field of view of the user. Hence, in such an embodiment, two communicatively coupled head-up displays may be provided.

In an embodiment, the communication link is bidirectional.

In an embodiment, the display arrangement comprises a mouthpiece for the rebreather having a first mounting provision at which the first mounting element of the first communication partner device (such as a head-up display) is mounted or mountable (for instance, the mounting may involve forming a screw connection, a clamping connection, a snap-fit connection or the like between the mounting provision and the mounting element). The mouthpiece provides a suitable position for fastening the first communication partner device without disturbing a user during use of the rebreather and at the same time allowing the user to keep the second communication partner device configured as a head-up display always in the visible range.

In an embodiment, the display arrangement comprises a user mask for the diving apparatus (particularly rebreather) having a second mounting provision at which the second mounting element of the head-up display is mounted or mountable. The user mask is another appropriate position for fastening the head-up display without disturbing a user during use of the rebreather and at the same time allowing the user to keep the head-up display always in the visible range.

In an embodiment, the first communication partner device when being configured as further head-up display comprises at least one light source, particularly at least one light emitting diode, for visually displaying the further data. Such a light emitting diode may be a dual-color or a multi-color light emitting diode allowing to indicate many different operation states of the rebreather (or other kind of diving apparatus) with one and the same light emitting diode. Alternatively, the further head-up display may comprise a two-dimensional display area, particularly an OLED (organic light emitting diodes) array or a liquid crystal display (LCD). It is however preferred that the further head-up display is very simple in terms of hardware requirements and ease of operation.

In an embodiment, the head-up display comprises a two-dimensional display area, particularly an OLED array or a liquid crystal display, for visually displaying the data. Thus, it is possible to display to a user a large variety of different kind of data.

In an embodiment, the wireless communication link is configured for transmitting the light signal as infrared light or visible light. The rebreather data transmission system may operate with light pulses being infrared light pulses. Alternatively, the rebreather data transmission system may operate with light pulses being visible light pulses. The optical and infrared wavelength ranges have turned out as highly suitable for transmitting data in water or related media.

In an embodiment, the wireless communication link is configured for transmitting a pulsed light signal, particularly with a carrier frequency in the range between about 25 kHz and about 500 kHz. Thus, a rebreather data transmission system is provided, wherein said light pulses use a carrier frequency between preferable 25 kHz and 500 kHz. Transmission of data with such a carrier frequency has turned out as highly failure robust.

In an embodiment, the wireless communication link comprises a light emitting element, particularly an infrared emitter, configured for emitting the light and being located at the first communication partner device , and a light sensitive element, particularly an infrared sensor, configured for sensing the light and being located at the second communication partner device . Both elements may be infrared diodes.

In an embodiment, the head-up display of the second communication partner device comprises a plano-convex lens having a planar side being exposed to an environment, particularly water, of the display arrangement and having a convex surface being sealed against the environment. Even when exposing the planar surface of this lens to water the transmission of an image to be displayed from a two-dimensional display to a user viewing onto the planar surface of the lens will not suffer from optical artifacts.

In an embodiment, the head-up display of the second communication partner device comprises a concave lens (for instance having two opposing concave surfaces) arranged in an optical path upstream of the plano-convex lens. Hence, a light propagation direction may be from a display array via the concave lens towards the plano-convex lens.

In an embodiment, the head-up display of the second communication partner device comprises a reflective member arranged between the concave lens and the plano-convex lens. By providing a single reflection mirror the optic path may be folded only once and may therefore be kept simple and compact.

In an embodiment, the wireless communication link is an unidirectional communication link. By taking this measure, the communication link may be embodied with smaller hardware and software effort. Alternatively, a communication link may be provided, where the data transmission is bidirectional. In such an embodiment, the transmitter may also be configured as a transceiver (capable of transmitting signals and of receiving signals).

In an embodiment, the displayed data is diving operation relevant data, particularly rebreather operation relevant data, more particularly at least one of the group consisting of data indicative of the status of the rebreather, data indicative of readings of partial oxygen pressure sensors, data indicative of readings of tank pressure sensors, data indicative of readings of a carbon dioxide sensor, data indicative of a status of a breathing gas filter cartridge, and data indicative of a quantity of the transmitted second data. However, other data may be displayed as well.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Figure 1 illustrates an explosive view of a mouthpiece of a rebreather according to an exemplary embodiment of the invention.
Figure 2 illustrates the mouthpiece of Figure 1 in an assembled state.
Figure 3 illustrates the mouthpiece of Figure 1 in an open circuit mode.
Figure 4 illustrates the mouthpiece of Figure 1 in a closed circuit mode.
Figure 5 illustrates a countering arrangement and a transportation arrangement of the rebreather.
Figure 6 illustrates a carbon dioxide filter mounted between two counterlung sections of an integrally formed countering of the rebreather of Figure 5.
Figure 7 illustrates the transportation arrangement of the rebreather in an opened state in which rebreather components are exposed to an environment.
Figure 8 illustrates the transportation arrangement of the rebreather in a closed state ready for transportation in which rebreather components are shielded with regard to an environment by a cover shell.
Figure 9 illustrates a display arrangement of the rebreather having two head-up displays in the visible range of the user.
Figure 10 illustrates the optical data transmission path within one of the head up displays of the display arrangement of Figure 9.
Figure 11 illustrates a carbon dioxide filter mounted within a counterlung to thereby separate two counterlung sections within an interior volume of the integrally formed counterlung according to an exemplary embodiment of the invention.
Figure 12 illustrates a front view of a mouthpiece of a rebreather according to another exemplary embodiment of the invention.
Figure 13 illustrates the mouthpiece of Figure 12 in a side view.
Figure 14 illustrates the mouthpiece of Figure 12 in a further side view.
Figure 15 illustrates a cross-sectional view of the mouthpiece of Figure 12 along a line A-A in a closed circuit switch state.
Figure 16 illustrates another cross-sectional view of the mouthpiece of Figure 12 along a line B-B in a closed circuit switch state.
Figure 17 illustrates a front view and a cross-sectional view along a line C-C of the mouthpiece of Figure 12 in an open circuit switch state.
Figure 18 illustrates two three-dimensional views of the mouthpiece of Figure 12.
Figure 19 shows a front view of an overpressure valve of the mouthpiece of Figure 12 to Figure 18 in an open circuit switch state.
Figure 20 shows a cross-sectional view along a line A-A of the overpressure valve of Figure 19 in the open circuit switch state.
Figure 21 shows a front view of the overpressure valve of Figure 19 in a closed circuit switch state.
Figure 22 shows a three-dimensional view of the overpressure valve of Figure 19 in the open close search mode.
Figure 23 illustrates a three-dimensional view of a disassembled state of a carbon dioxide filter to be mounted between two counterlung sections of an integrally formed counterlung of a rebreather of the type shown in Figure 5.
Figure 24 illustrates a cross-sectional view of the carbon dioxide filter of Figure 23 mounted to the counterlung.

The illustration in the drawing is schematical. In different drawings, similar or identical elements are provided with the same reference signs.

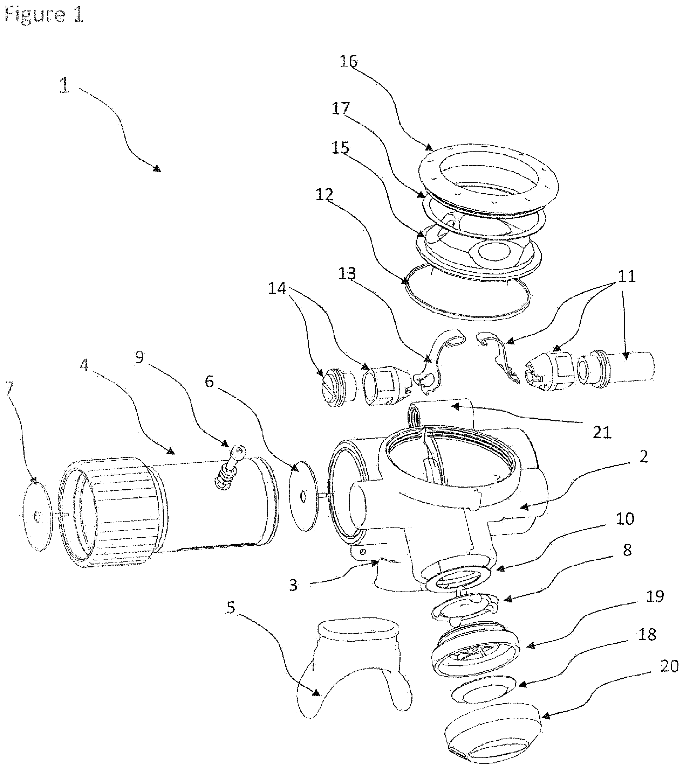

**Figure 1** illustrates an explosive view of a mouthpiece 1 of a rebreather according to an exemplary embodiment of the invention.

The mouthpiece 1 for the rebreather is selectively operable in a closed circuit (CCR) mode or in an open circuit (OC) mode. The mouthpiece 1 comprises an inhale direction valve 6, an exhale direction valve 7, an automatic diluent valve 11, and an overpressure valve formed of multiple components 8, 9, 22, 18.

A switching barrel 4 is accommodated in a housing formed of housing parts 2, 3 and is switchable by a user by turning barrel 4 between a closed circuit switch state for operating the mouthpiece 1 in the closed circuit mode and an open circuit switch state for operating the mouthpiece 1 in the open circuit mode.

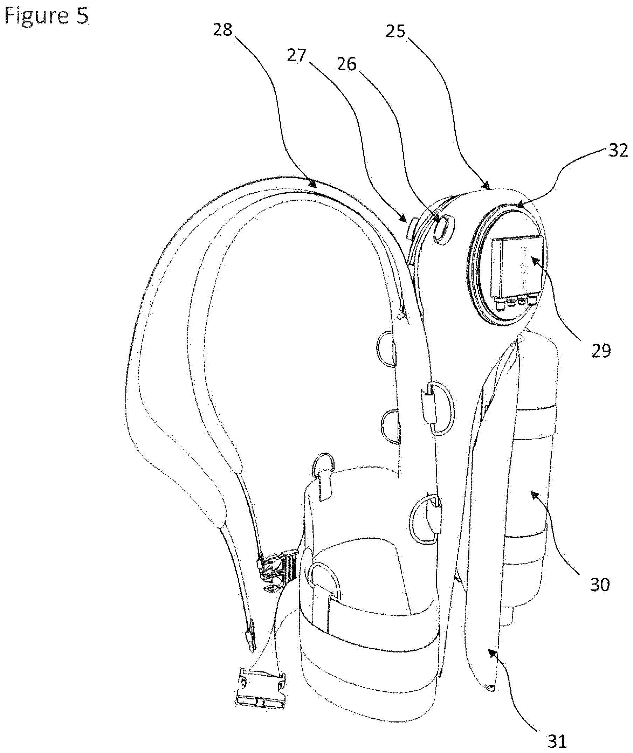

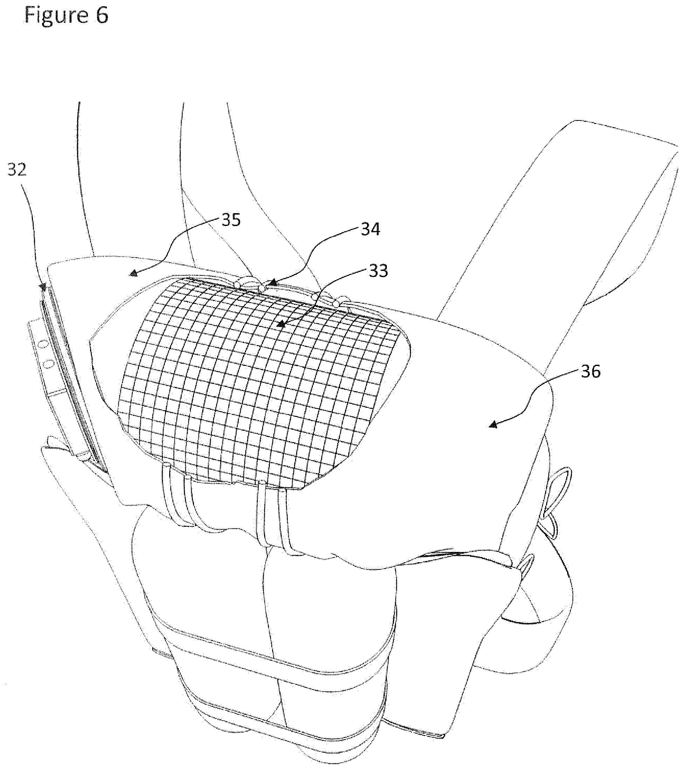

In the closed circuit switch state, a user is enabled to inhale breathing gas via the inhale direction valve 6 and to exhale breathing gas via the exhale direction valve 7. In the closed circuit switch state, a breathing gas flow loop is formed between a counterlung 25 in cooperation with a carbon dioxide filter cartridge 33 (compare Fig. 5 and Fig. 6) and the hollow volume within the mouthpiece 1. More precisely, the inhale direction valve 6 is connected to an inhale counterlung section 35 by a hose connection or the like (not shown). Correspondingly, the exhale direction valve 7 is connected to an exhale counterlung section 36 by another hose connection or the like (not shown). A user may inhale breathing gas from inhale counterlung section 35 via the inhale direction valve 6. A user may exhale carbon dioxide enriched breathing gas towards the exhale counterlung section 36 via the exhale direction valve 7. When passing from the exhale counterlung section 36 to the inhale counterlung section 35, carbon dioxide is at least partially removed from the breathing gas.

The overpressure valve 8, 9, 22, 18 serves as a protection against overpressure in the loop in the closed circuit mode and opens when an overpressure in the mouthpiece 1 exceeds a first threshold pressure of for instance 20 mbar. The automatic diluent valve 11 may be connected to a breathing gas supply bottle (not shown) and provides a diluent gas in the closed circuit mode when a negative pressure in the loop exceeds a second threshold pressure of for instance -30 mbar.

In the open circuit switch state, the flow path between inhale direction valve 6 and exhale direction valve 7 is isolated from the mouthpiece 1 and is therefore deactivated. In the open circuit switch mode, the user is enabled to inhale breathing gas via the automatic diluent valve 11 from the breathing gas supply bottle (not shown). In the open circuit mode, a user is further enabled to exhale breathing gas via the overpressure valve 8, 9, 22, 18 (particularly the exhaust valve part 18 thereof), since the overpressure valve 8, 9, 22, 18 is deactivated in the open circuit switch state so as to not serve as a protection against overpressure in this operation mode. In the open circuit mode, the automatic diluent valve 11 provides the diluent gas or breathing gas if a negative pressure in the mouthpiece 1 exceeds a third threshold pressure of for instance
-3 mbar, therefore having a significantly smaller absolute value than the second threshold pressure. In other words, the negative threshold pressure beyond which dilution gas is delivered to the mouthpiece 1 has an absolute value which is higher in the closed circuit mode as compared to the open circuit mode.

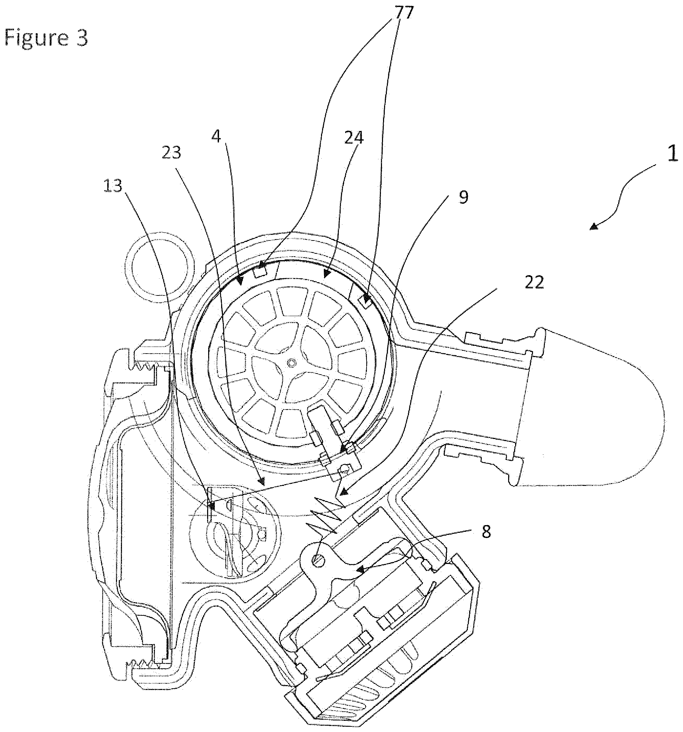

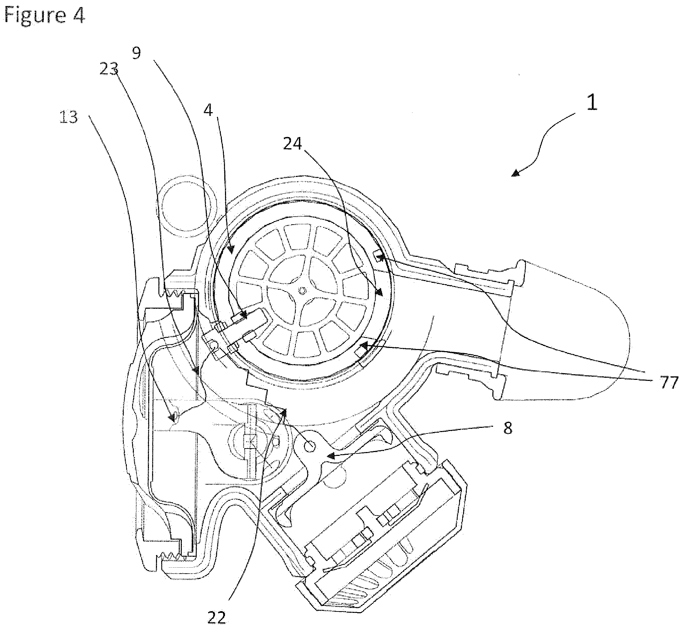

The mouthpiece 1 includes the following components: The housing is formed by two parts which are bonded together - the left 2 and the right half housing 3. The barrel 4 sits inside the housing and is used to switch the mouthpiece 1 in CCR mode (see Figure 4) or OC mode (see Figure 3). In CCR mode the diver inhales through the rubber mouth connection piece 5. Gas enters the mouthpiece 1 through the inhale direction valve 6 and exits through the exhale direction valve 7. The overpressure valve is formed by a plate 8 which is connected to a lever 9 on the barrel 4 with a tension spring 22. In CCR mode said plate 8 is pulled against a sealing 10. The spring constant and the length of the spring are selected in order to achieve an opening pressure threshold of preferable 15 to 40 mbar, in the present example 20 mbar. Downstream second stage valve or additional diluent valve (ADV) 11 is used as ADV in CCR mode and delivers gas in OC mode. A lever of the downstream second stage valve 11 is actuated by a diaphragm 12. In order to achieve a higher cracking pressure in CCR mode (see Figure 4), preferable -15 to -50 mbar and in the present example -30 mbar, a further spring loaded lever 13, which is part of a dummy downstream valve 14 without gas supply, applies an additional force to the diaphragm 12.

A purge button 15 is used to clear the mouthpiece 1 from water in OC mode and to add additional diluents in CCR mode. The purge button 15 and the diaphragm 12 are held in place with a threaded ring 16 and a washer 17.

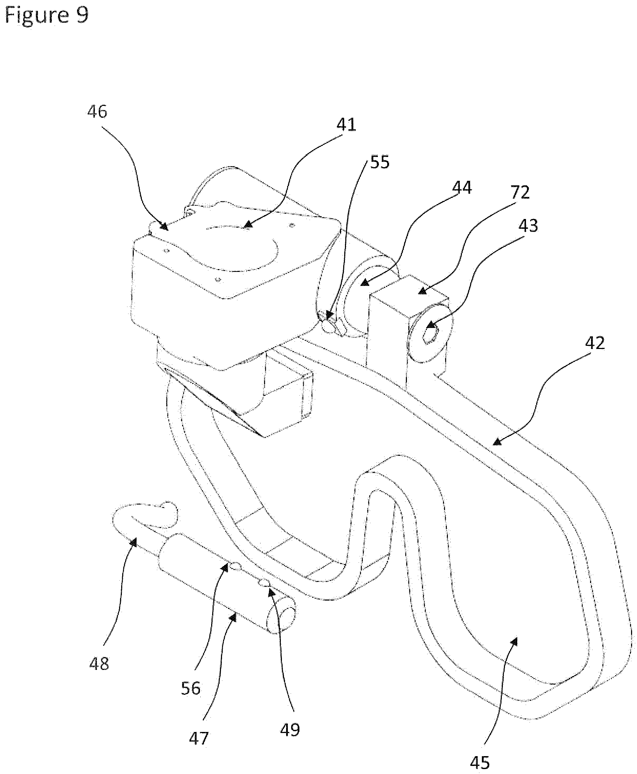

In OC mode (see Figure 3), the tension spring 22 is discharged, thus the plate 8 of the overpressure valve does not seal anymore. In OC mode the diver exhales through the exhaust valve 18 which is mounted with a support 19. A cap 20 protects the exhaust valve 18 from dirt. A support 21 for mounting a primary head-up display 47 (see Figure 9) is part of the left half housing 3 and serves as a mounting provision at which a corresponding mounting element of the head-up display 47 is mountable.

In OC mode the further lever 13 of the dummy valve 14 is pulled inside the housing 2, 3 with a nylon wire 23 (see Figure 3 and Figure 4).

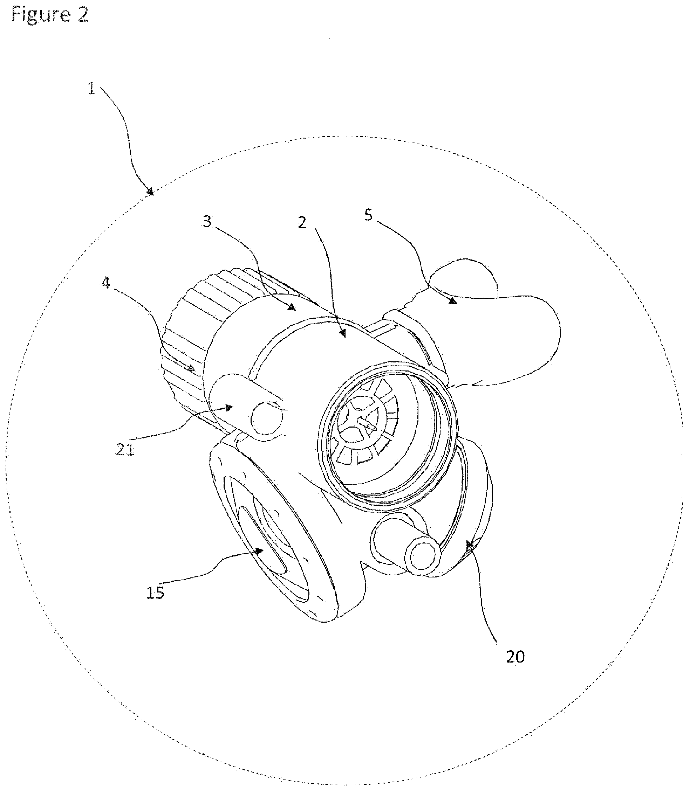

**Figure 2** shows the mouthpiece 1 of Figure 1 in an assembled state.

**Figure 3** shows the mouthpiece 1 in the open circuit mode. The nylon wire 23 is under tension. The spring 22 is in a non-elongated state.

**Figure 4** illustrates the mouthpiece 1 in a closed circuit mode. The nylon wire 23 is not under tension. The spring 22 is in an elongated state.

In CCR mode breathing gas enters the barrel 4 through the inhale valve 6, and reaches the rubber mouth connection piece 5 through an opening 24 in the barrel 4. In OC mode this opening 24 is closed by sealing it against the housing 2, 3 of the mouthpiece 1 with an O ring 77.

As can be taken from Figure 1 to Figure 4, a mouthpiece 1 is provided, which includes the following functions:
- CCR mode
- OC mode
- Integrated ADV 11
- Two different cracking pressures for OC mode and ADV 11
- Integrated overpressure valve 8, 9, 22, 18 for the closed circuit mode
- Primary LED based head-up display 47
- Purge button 15 similar like the one on an OC second stage regulator, which also serves as manual diluents valve.

The barrel 4 is located in between the mushroom type direction valves 6, 7, which allows switching the mouthpiece 1 from OC to CCR mode. In front there is the diaphragm 12, which actuates downstream valve 11. Below there is exhalation valve or exhaust valve 18.

In CCR mode the diver breaths on the closed circuit loop through the mushroom valves 6, 7. When the diver is descending, additional diluent gas is required which is delivered though the ADV 11, where the diaphragm 12 actuated the downstream valve. In order to increase the cracking pressure, second dummy downstream valve 14 is integrated, which is not supplied with gas, and serves the single purpose of exciting a counterforce on the diaphragm 12 to increase the cracking pressure. The ADV cracking pressure can be easily adjusted by compressing the spring in the dummy valve 14 or by changing the spring of the dummy valve 14 to one with a different spring constant and/or different length. In CCR position, the mouthpiece 1 also acts as overpressure valve, which is formed by a plate 8 which is pulled to a seat by a tension spring 22. The tension spring 22 is selected to that the opening pressure of the overpressure valve is preferable between 10 and 50 mbar.

In OC mode the barrel 4 is rotated and the loop is isolated from the mouthpiece 1, or more precisely from the mouth connection piece 5. By rotating the barrel 4, the tension on the plate 8 of the overpressure valve is released and the overpressure valve opens. The diver exhales through the exhalation valve 18 into the water. Additionally the dummy downstream valve 14 is pulled inside the mouthpiece 1, more precisely into the housing 2, 3 of the mouthpiece 1. In that position, it excerpts no force anymore on the diaphragm 12 and the cracking pressure in OC mode is preferable between 0.1 and 5 mbar.

The mouthpiece 1 is designed in a way, so that its two-part housing 2, 3 can be injection molded with a simple and cost efficient mold. By splitting the mouthpiece housing 2, 3 in one right and one left part, which are then bonded together, both parts can be molded in a simple two parts mold without costly purges and punches.

**Figure 5** and **Figure 6** illustrate a counterlung arrangement and a part of a transportation arrangement of the rebreather of which a corresponding mouthpiece 1 is shown in Figure 1 to Figure 4.

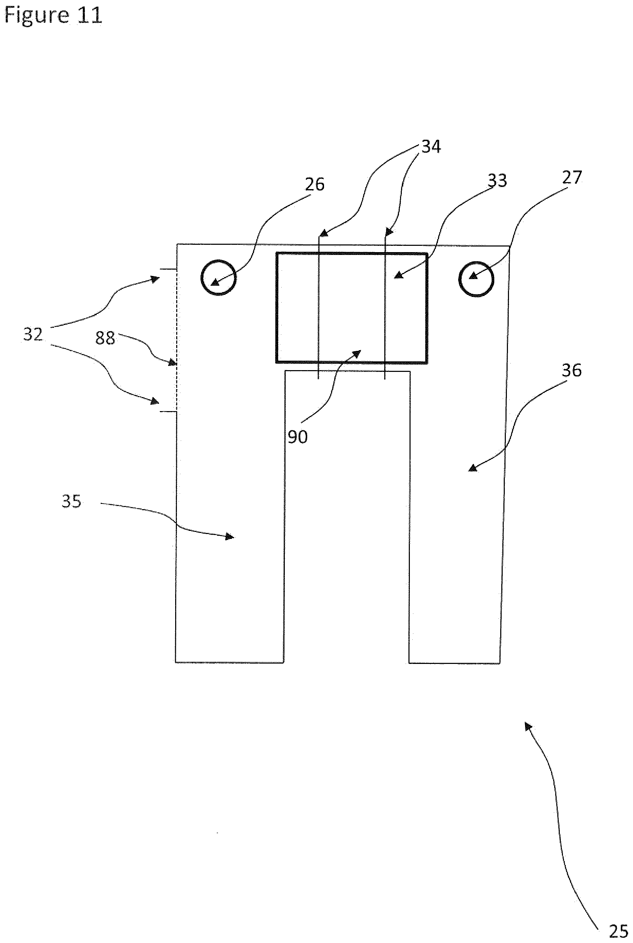

Figure 5 shows a countering 25 which is fabricated from polyurethane or polyvinylchloride coated fabric. Preferably, HF (high frequency) welding is used for the fabrication of the counterlung 25. The mouthpiece 1 is connected with hoses (not shown) to an exhale port 27 and an inhale port 26 of the counterlung 25. A combined sensor and control unit 29 is placed on the inhale side of the counterlung 25 in a ring 32, which is mounted on the counterlung 25, preferable with HF welding. When the sensor and control unit 29 is removed, a CO₂ filter cartridge 33 (see Figure 6) can be fully inserted via a selectively openable or closable access opening (see reference numeral 88 in Figure 11) into the counterlung 25. Two tanks 30, one for diluent and one for O₂ are mounted on the back. The diver carries the rebreather with a harness 28. Figure 5 also shows a buoyancy compensator 31.

Figure 6 illustrates the carbon dioxide filter cartridge 33 of the counterlung arrangement of the rebreather of Figure 5. In the illustration of Figure 6, a part of the cover fabric is removed so as to visually expose the breathing gas filter cartridge 33 inserted via ring 32 into an interior volume of the counterlung 25. However, when the breathing gas filter cartridge 33 is inserted into the counterlung 25, it is no more visible from outside unless the material of the counterlung is optically transparent (in fact, the material of the counterlung 25 may be optically transparent, semi-transparent or opaque). Further details can be taken from Figure 11.

More specifically, the counterlung arrangement comprises the integrally formed counterlung 25 formed as a single piece which in turn comprises an inhale counterlung section 35 to be connected to an inhale side of the mouthpiece 1 and an exhale counterlung section 36 to be connected to an exhale side of the mouthpiece 1. A filter accommodation section is formed as an interior hollow volume within the bag-like counterlung 25 between the inhale counterlung section 35 and the exhale counterlung section 36 and is configured for accommodating the breathing gas filter cartridge 33 such as a carbon dioxide filter cartridge. The breathing gas filter cartridge 33 is configured for filtering breathing gas flowing between the exhale counterlung section 36 and the inhale counterlung section 35 when being accommodated in the filter accommodation section. The counterlung 25 and the breathing gas filter cartridge 33 are designed to precisely correspond to one another so that the breathing gas filter cartridge 33 is sealingly clamped between the exhale counterlung section 35 and the inhale counterlung section 36 in an interior volume of the counterlung 25 so as to be circumferentially surrounded by counterlung material. This can be achieved by configuring the described components so that the filter accommodation section between the elastic counterlung sections 35, 36 is slightly smaller than an extension of the rigid breathing gas filter cartridge 33 so that, when a user fills the previously empty filter accommodation section between the counterlung sections 35, 36 by inserting the breathing gas filter cartridge 33 a clamping force exerted by the counterlung 25 holds the breathing gas filter cartridge 33 in a sealing manner in between without the necessity for a further separate fastening mechanism apart from the clamping fastening.

A counterlung may be understood as a buffer volume (such as a buffer volume delimited by a plastic bag on the like) into which a user may exhale and from which a user may inhale. Such a counterlung may be in accordance with the European standard EN 14143.

However, according to an exemplary embodiment of the invention, the counterlung 25 is internally functionally separated into the inhale counterlung section 35 and the exhale counterlung section 36 which are however structurally integrally formed from a single bag body divided into two sections when the carbon dioxide filter 33 is inserted.

Figure 6 shows the rebreather from the top with open counterlung 25 to illustrate how the CO₂ filter cartridge 33 is placed in between the inhale side 35 and the exhale side 36 of the counterlung 25. The filter cartridge 33 is inserted through the ring 32 on the inhale side of the counterlung 25 into an interior hollow volume of the counterlung 25. After insertion, the filter cartridge 33 is circumferentially fully surrounded by the counterlung 25 and is, after closing the opening through the ring 32, no more visible from outside the counterlung 25 unless the counterlung material is optically transparent. The filter cartridge 33 is held in place and sealed against the counterlung 25 with a flexible rubber band 34 to avoid gas bypassing the filter 33 from the inhale to the exhale side.

In conventional approaches, a counterlung design for recreational rebreathers comprises two separate counterlungs - one inhale counterlung and one exhale counterlung with a scrubber canister in between. Some systems have only one counterlung, but for performance reasons of the scrubber, a twin counterlung is better, as the gas flow speed through the scrubber is just half. In all recreational and technical rebreathers there is a separate scrubber canister. Some professional closed circuit rebreathers for military and special forces applications have a scrubber canister, which is integrated into a counterlung. These systems however feature only a single counterlung and still require a scrubber housing.

Instead of having two separate counterlungs, a scrubber housing and hose connections in between, the embodiment of the invention shown in Figure 5 and Figure 6 shows a combined inhale and exhale counterlung 25, which can be fabricated from one piece. The counterlung 25 is equipped with an opening, which allows insertion of a CO₂ filter cartridge 33, preferable a single use pre packed filter. The filter 33 is inserted in the filter accommodation section in between the inhale side and exhale side of the counterlung 25. Preferably, such a counterlung 25 is made from HF weldable polyurethane of PVC foil. In these cases the section in between the inhale side and exhale side of the counterlung is realized with a slightly larger circumference than the one of the CO₂ filter. Once the filter 33 is inserted, a rubber strap 34 is mounted around this section, compresses the counterlung foil and seals the counterlung 25 to the filter 33. It is important to achieve a good sealing, in order to prevent gas bypassing the CO₂ filter from the inhale to the exhale side, as this could increase the CO₂ level in the inhaled gas. The main advantages of this design are:
- The counterlung 25 can be fabricated as one piece.
- The amount of connections is very small.
- No separate scrubber housing is necessary.
- Amount of possible failure points, like leaking O-rings in hose connectors, are reduced.
- The counterlung 25 can be fabricated at low cost.

Neoprene rubber can be used as alternative material for the counterlung 25. Then the section in between the inhale and exhale side of the counterlung 25 is smaller than the circumference diameter of the materials to be able to realize a proper sealing without an additional rubber strap 34.

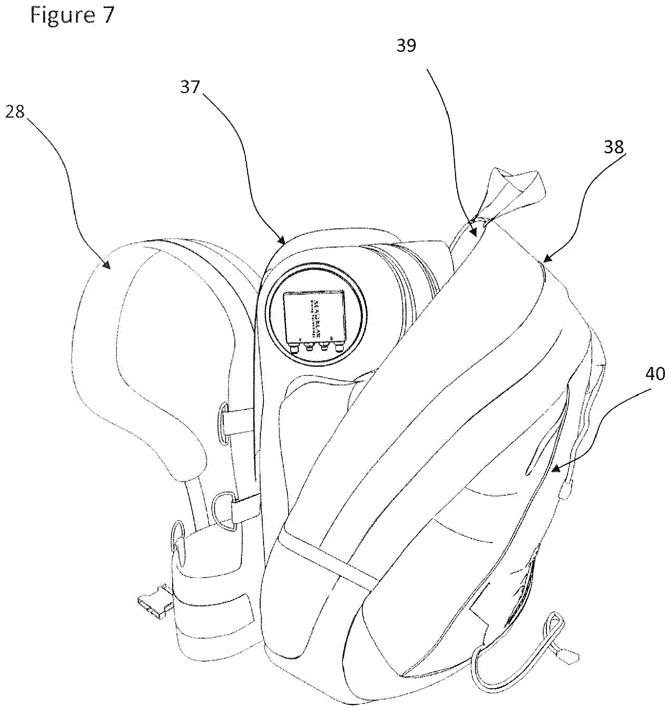

**Figure 7** illustrates the transportation arrangement of the rebreather in an opened state.

The transportation arrangement or transport device comprises the harness 28 configured to be wearable by a user by fastening the harness 28 at an upper part of the body of the user. Rebreather components (such as mouthpiece 1, counterlung 25, gas bottle 30, carbon dioxide filter cartridge 33, first head-up display 47, second head-up display 41) are mounted on the harness 28 so as to be arranged at the back of the user when wearing the harness 28 (note that some of the components, particularly mouthpiece 1, mask and head up displays 47, 41 may be detached after use from their corresponding in-use positions in front of the user's body and stored at the harness 28). A rebreather component cover or shell 38 is configured for covering at least a part of the rebreather components 1, 25, 30, 31, 33, 41, 47 when mounted on the harness 28. The harness 28 and the rebreather component cover 38 are provided with matching fastening elements embodied as a zipper 39 and being configured so that the rebreather component cover 38 is selectively fastenable to the harness 28 to thereby cover at least a part of the rebreather components 1, 25, 30, 31, 33, 41, 47 for transportation or detachable from the harness 28 to thereby expose the rebreather components 1, 25, 30, 31, 33, 41, 47 for subsequent use of the rebreather (for instance for diving).

Figure 7 shows how the rebreather can be transformed into a backpack. After the mouthpiece 1 and the hoses are disconnected from the inhale and exhale port of the counterlung 25, shell 38 in the form of a backpack can be mounted, preferable with the zipper 39 (or with a Velcro), on the harness 28. In this way, the rebreather is transformed into a backpack, which allows easy transportation and protection of the rebreather. All parts of the rebreather can be stored inside. The shell 38 has a circumferencing zip 39. The counterpart of the zip 39 is on the harness 28. The shell 38 is also equipped with pockets 40 to enable quick access to personal belongings.

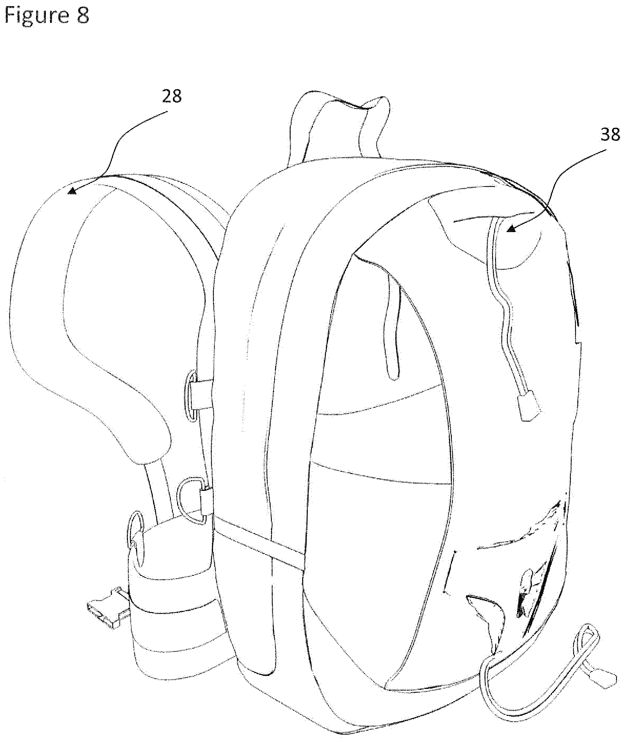

**Figure 8** illustrates the transportation arrangement of the rebreather in a closed state. In other words, Figure 8 shows the shell 38 completely mounted on the harness 28.

**Figure 9** illustrates a display arrangement of the rebreather.

The display arrangement comprises first or primary head-up display 47 for visually displaying first data to a user and having a first mounting section 43 to be mounted at a first position of the rebreather in a field of view of the user, and a second or secondary head-up display 41 for visually displaying second data to the user and having a second mounting section to be mounted at a second position of the rebreather in the field of view of the user.

A wireless communication link 49, 55 (formed of infrared emitting diode 49 of the primary head up display 47 and an infrared sensitive diode 55 of the secondary head up display 41) is configured for transmitting the second data in a wireless manner via a light signal which is indicative of the second data from the first head-up display 47 to the second head-up display 41.

Figure 9 shows the primary head-up display 47 which is typically located on the mouthpiece 1 and the secondary head-up display 41 which is mounted with support or mounting section 43 on a corresponding mounting provision 72 of a frame 42 of a diving mask. The frame 42 holds a visor 45 of the diving mask.

The primary head-up display 47 is connected to an electronic control module (not shown in Figure 9) with a cable 48. The primary head-up display 47 is further equipped with a dual color LED 56 in addition to the infrared LED 49. The infrared LED 49 is arranged in free line of sight with the infrared receiver 55 on the secondary head-up display 41. A ball joint 44 is located between the secondary head-up display 41 and the support 43 to enable an adjustment of the secondary head-up display 41 in respect to the diver's eye. Further it allows turning the head-up display 41 up outside the diver's field of view when it is not used. The secondary head-up display 41 is equipped with an USB port 46 for data download and recharging the battery.

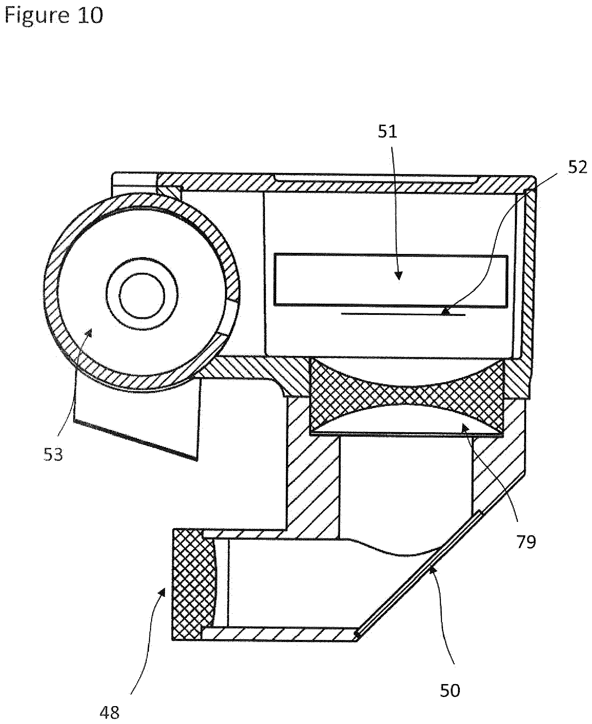

**Figure 10** illustrates the optical data transmission path of the display arrangement of Figure 9.

More precisely, Figure 10 shows the design of the optical path of the secondary head-up display 41. It consists of one plano-convex lens 48 located in front of the visor 45 of the diving mask. The plane side of the plano-convex lens 48 is in contact with the water. A concave lens 79 is mounted in front of electronic module 51 with the micro OLED display 52. The optical path is folded with a single reflective surface mirror 50. Reference numeral 53 is a compartment for a Li-ion rechargeable battery.

The primary head-up display 47 is hence mounted on top of the mouthpiece 1. It consists of two diodes 49, 56, one bicolor emitting red or green diode 56 and another one emitting infrared (diode 49). The bicolor LED 56 serves as simple to understand warning device. The following functions can be realized.

Alarm level 0: One short green flash, preferable 50 to 200 ms long, in a period of preferable 1 to 3s: in this state the HUD 47 indicates that the rebreather is correctly functioning and that the diver can breathe from the mouthpiece 1 in CCR position

Alarm level 1: Two short red flashes, preferable each 50 to 200 ms long, with a pause in between of preferable 50 to 200 ms, in a period of preferable 1 to 2s: in this state the HUD 47 indicates a minor problem, the diver still can breathe from the mouthpiece 1 in CCR position, but should read immediately the handset or the secondary HUD 41 to determine the problem. Such am alarm is indicated for example when the tank pressure of O₂ and diluents is below a certain threshold, preferable in between 10 and 70 bar.

Alarm level 2: Continuous short red flashes, preferable each 50 to 200 ms long, with a frequency of preferable 2 to 10 Hz. This alarm indicates a life threatening rebreather failure, such as for example a pO₂ outside life sustaining limits. The diver should respond to such an alarm with an immediate switch of the mouthpiece 1 to OC mode.

Alarm level 3: no red, no green or continuous red, green or amber light - this state should not appear during normal operation. It indicates a software or hardware failure and the diver's immediate response should be to switch the mouthpiece 1 to OC mode and abort the dive.

The primary HUD 47 is also equipped with the infrared diode 49. This diode 49 serves to send dive relevant data to the HUD 41 mounted on top of the diver's mask.

In diving, wireless data transmission is usually achieved with electromagnetic communication, which is based on am electromagnetic carrier with a frequency of 5-32 kHz. Such a low frequency allows only a small data rate and is not antimagnetic, which may be a problem when the rebreather is used for instance for military applications.

According to an exemplary embodiment of the invention, it is possible to use a one directional infrared link, in order to transmit rebreather data from the primary HUD 47 to the secondary HUD 41.

Therefore, the second infrared LED 49 is placed next to the dual color LED 56 on the primary HUD 47.

It allows wireless transmission of the rebreather data with infrared light. For the implementation a UART asynchronous serial communication protocol with 2400 BAUD can be chosen. When a logical "1" is transmitted, the infrared diode 49 emits light for 0.42 ms with a frequency of 38kHz. When transmitting a logical "0" no light is emitted. The data package is transmitted in predefined intervals, preferable 0.5 to 2 times per s, and includes:
- Status of the rebreather
- Readings of pO₂ sensors
- Readings of the tank pressure sensors
- Reading of a CO₂ sensor
- Scrubber status
- Checksum

The secondary head-up display 41 is mounted on top of the diver's mask. It features a 96x64 pixel OLED display. A small infrared receiver is located on the battery housing, and used to demodulate the infrared data. In this position there is a clear line of sight to the infrared LED 49 on the primary HUD 47, thus data reception is possible.

The secondary HUD 41 consists of the following components:
- Microcontroller Atmel ATmega 644 P or Atmel AVR32 UC3B0256 operated at preferably 8 to 66 MHz
- Pressure / depth / temperature sensor digital pressure sensor from Intersema, Switzerland
- Tilt compensated digital compass consisting of a three axial magnetometer and a three axial accelerometer combined three axial magnetometer and three axial compass - LSM303, ST Microelectronics
- 96x64 pixel color display, 0,96" OLED, Densitron
- Step up voltage converter to generate a 14V supply for the OLED display
- 64 MBit FLASH memory Dataflash, Atmel
- Infrared receiver
- Rechargeable Li Ion battery, with preferable 3.5 to 4.2 V and 600 to 1800 mAh capacity
- Li ion battery charger circuit, Maxim MAX-1555

A single convex lens magnifier near eye display works in general well, but delivers a largely magnified virtual image. Especially when using color displays this is disturbing, as one will rather see three separate color pixels instead of one merged color pixel. In general, a high magnification is also not required.

In order to overcome the problem of the high magnification, an optical path is implemented which consists of three parts: A magnifying plan-convex lens 48, with a focal length of 30 - 150mm, preferable 50-60mm, forms the window of the device later referred to as front lens. The planar side of the lens 48 is in contact with water and the curved side is inside the housing. A proper sealing of the lens 48 is advantageous. One can use mechanical sealings like O-ring sealing or gluing. In the case of a plastic lens and a plastic housing one may perform ultrasonic or chemical welding to bond the lens to the housing.

A bi-concave lens 79 is introduced into the optical path and situated in front of the display 52, preferable in a distance of 5 to 15 mm. The focal length is between -10 and -50 mm, preferable -20mm. The lens diameter is between 10 and 40mm, preferable between 13 and 25 mm.

The optical path is folded by an angle in between 50 and 150°, preferable 90°, with single surface mirror 50. Single surface mirror 50 is advantageous to avoid ghost images.

With that arrangement of two lenses 48, 79 and the display 52, an optical magnification ratio of approximately 1:1 can be achieved, which turned out to be optimal. The distance between the front lens 48 and the display 52 is calculated to achieve a virtual image in a distance of 20cm to infinity, preferable 100 cm.

**Figure 11** illustrates a cross-sectional view of a counterlung arrangement in which a carbon dioxide filter 33 is mounted between two counterlung sections 35, 36 in an interior volume of an integrally formed counterlung 25 according to an exemplary embodiment of the invention. In the cross-sectional view of Figure 11, the counterlung 25 is U-shaped. Just for the purpose of explanation, the counterlung 25 may have a shape similar to that of jeans with closed ends of the legs and a closed waist to thereby form kind of bag. Each of the legs then corresponds to one of the counterlung sections and the waist may correspond to the filter accommodation section.

The single piece counterlung 25 has one inhale side 35 and one exhale side 36. In CCR mode the diver breathes from the inhale lung 35 through the inhale port 26 on the counterlung 25, an inhale hose (not shown) which connects port 26 with the inhale side of the mouthpiece 1, and the inhale valve 6 in the mouthpiece 1. The diver exhales through the exhale valve 7 in the mouthpiece 1, through the exhale hose (not shown) that connects the exhale side of the mouthpiece 1 with the exhale port 27 on the exhale side 36 of the counterlung 25. Through a port such as a ring 32 (enclosing a selectively openable or closable access opening 88) a CO₂ filter cartridge 33 can be inserted in between the two sides of the counterlung 25. In this position, the CO₂ filter 33 divides the counterlung 25 into an inhale and exhale side.

The counterlung 25 is designed in a way, so that the filter cartridge 33 is sealed against the counterlung 25 to avoid gas bypassing the CO₂ filter 33 from inhale to exhale side.

Such a sealing can be achieved in different ways: If the counterlung 25 is fabricated from flexible and stretchable material, for instance neoprene rubber, the cross section of the counterlung 25 in the area of the CO₂ filter cartridge 33 is designed to be slightly smaller than the cross section of the filter 33, in order to achieve a proper sealing. If non stretchable material is used - like for instance polyurethane coated fabric then the cross section of the counterlung 25 will be designed slightly larger than that of the filter cartridge 33, to enable easy placement of the filter cartridge 33 inside the counterlung 25. Sealing can then be achieved with a strap 34 around the filter, for instance a strap made from flexible rubber.

In the counterlung arrangement of Figure 11, the counterlung 25 hence comprises the selectively openable or closable access opening 88 configured for providing an access for the insertion (or removal) of the breathing gas filter cartridge 33 into (or from) an interior volume of the counterlung 25. A for example locally narrowed section 90 of the interior volume is configured for clampingly holding the breathing gas filter cartridge 33 after its complete insertion into the interior volume via the access opening 88. The access opening 88 has slightly larger dimensions than the breathing gas filter cartridge 33 to simplify its insertion into the interior volume of the counterlung 25. A transition between the inhale counterlung section 35 and the accommodated breathing gas filter cartridge 33 is free of any connection member and/or a transition between the exhale counterlung section 36 and the accommodated breathing gas filter cartridge 33 is free of any connection member. The access opening 88 may be surrounded or delimited by a water-sealed closure such as a water-sealed zipper. Such a water-sealed closure may be opened to insert the filter cartridge 33 into an interior of the counterlung 25. The water-sealed closure may be closed to use the counterlung 25 and the filter cartridge 33 for supplying oxygen-rich breathing air to a diver when using the rebreather.

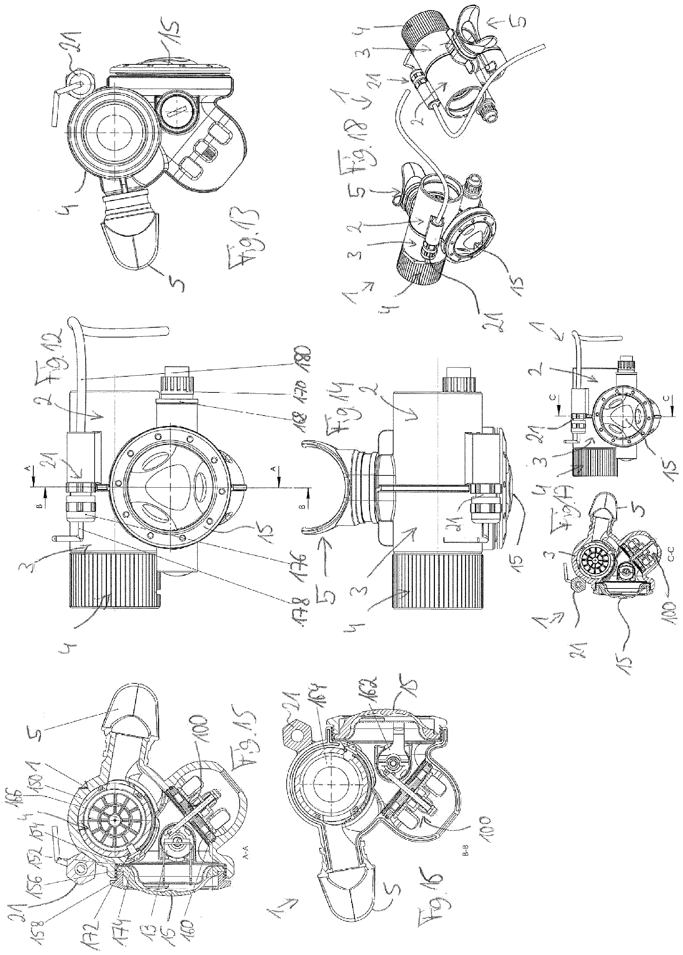

In the following, referring to Figure 12 to Figure 22, a mouthpiece 1 of a rebreather according to another exemplary embodiment of the invention will be explained. Reference is made to the above description, in particular the description of Figure 1 to Figure 4.

**Figure 12** illustrates a front view of the mouthpiece 1. **Figure 13** illustrates the mouthpiece 1 of Figure 12 in a side view. **Figure 14** illustrates the mouthpiece 1 of Figure 12 in a further side view. **Figure 15** illustrates a cross-sectional view of the mouthpiece 1 of Figure 12 along a line A-A in a closed circuit (CCR) switch state. **Figure 16** illustrates another cross-sectional view of the mouthpiece 1 of Figure 12 along a line B-B in the CCR switch state. **Figure 17** illustrates a front view and a cross-sectional view along a line C-C of the mouthpiece 1 of Figure 12 in an open circuit (OC) switch state. **Figure 18** illustrates two three-dimensional views of the mouthpiece 1 of Figure 12.

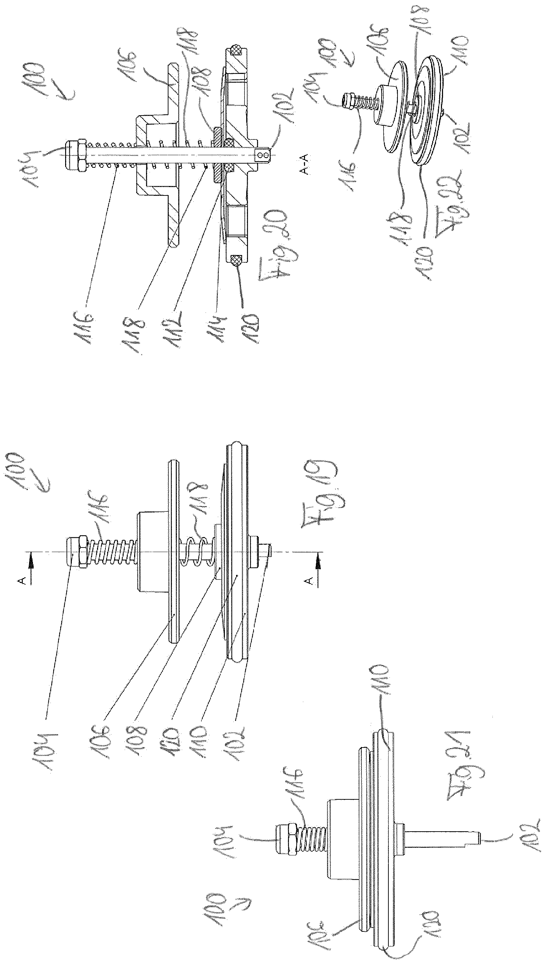

The CCR switch state and the OC switch state correspond to two different positions of an overpressure valve 100 (which is shown in Figure 19 to Figure 22 in further detail).

Among other features already described above, the mouthpiece 1 of Figure 12 to Figure 19 comprises an inner drum 150, a membrane 152, a membrane ring 154, a diving regulator cap 156, a diving regulator cap ring 158, spring ring 160, mouthpiece valve 162, membrane 164, outlet valve grid 166, washer 168, nut 170, sealing ring 172, actuator axle 174, cable glands 176, optical fiber 178, and head up display connection 180.

In the following, the overpressure valve 100 of the mouthpiece 1 of Figure 12 to Figure 18 will be explained in further detail. **Figure 19** shows a front view of the overpressure valve 100 in the OC switch state. **Figure 20** shows a cross-sectional view along a line A-A of the overpressure valve 100 in the OC switch state. **Figure 21** shows a front view of the overpressure valve 100 in the CCR switch state. **Figure 22** shows a three-dimensional view of the overpressure valve 100 in the OC switch mode.

The overpressure valve 100 comprises an axle 102, a prevailing torque nut 104, a plate 106, a distance member 108, a grid 110, a sealing ring 112, a further sealing ring 120, a membrane 114, a first biasing element 116 configured as a helical spring, and a second biasing element 118 also configured as a helical spring. Preferably, the first biasing element 116 has a higher spring constant than the second biasing element 118.

Figure 22 shows a three-dimensional view of the overpressure valve of Figure 19 in the open close search mode.

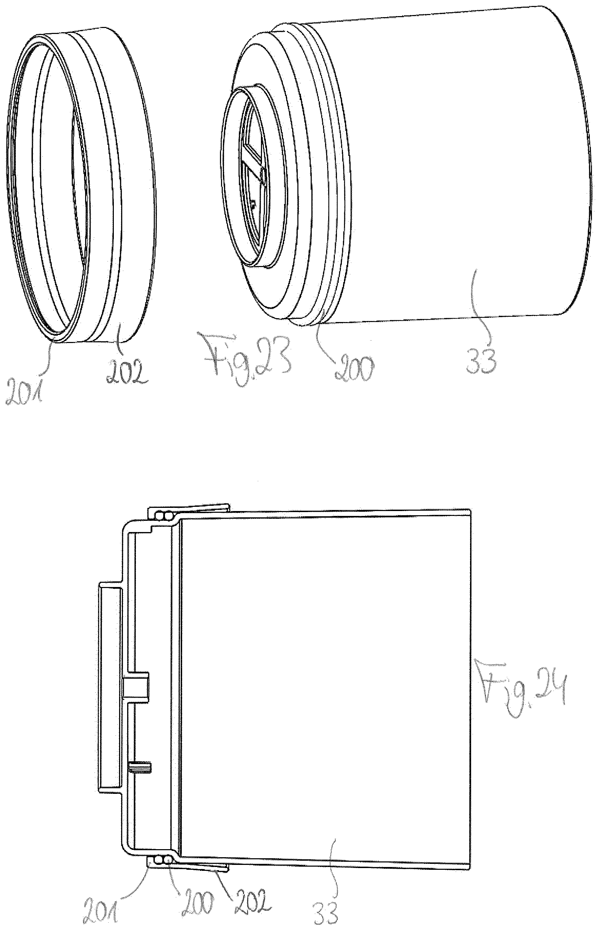

**Figure 23** illustrates a three-dimensional view of a disassembled state of a carbon dioxide filter 33 to be mounted between two counterlung sections 35, 36 (compare Figure 6) of an integrally formed counterlung 25 of a rebreather of the type shown in Figure 5. **Figure 24** illustrates a cross-sectional view of the carbon dioxide filter 33 of Figure 23 mounted to the counterlung.

In the present embodiment, the counterlung 25 and the breathing gas filter cartridge 33 are configured to match to one another so that the breathing gas filter cartridge 33 seals between the exhale counterlung section 36 and the inhale counterlung section 35 in an interior volume of the counterlung 25. More specifically, sealing of the breathing gas filter cartridge 33 against a counterlung wall in the filter accommodation section is achieved with a sealing 200 which is here embodied as an O-ring.

Figure 23 shows the CO₂ filter cartridge 33, a ring 201 which is mounted inside the counterlung, wherein a counterlung sealing surface 202 is preferable HF or ultrasonic welded, clamped or glued to the counterlung. Figure 24 shows the CO₂ filter cartridge inserted into the ring 201. Two O-rings 200 are used to seal the CO₂ filter cartridge 33 against the filter ring 201. Alternatively, sealings 200 can be used to seal the filter cartridge 33 directly against the counterlung section wall. By using sealings 200, a clamp 34 around the filter accommodation is not necessary and can be omitted in this embodiment.

## Claims

1. A display arrangement for a diving apparatus, the display arrangement comprising:
a first communication partner device (47) having a first mounting element to be mounted at a first position of the diving apparatus;
**characterized in that** the display arrangement further comprises
a second communication partner device configured as a head-up display (41) for visually displaying data to a user and having a second mounting element (43) to be mounted at a second position of the diving apparatus so that the head-up display (41) is in the field of view of the user;
a wireless communication link (49, 55) configured for transmitting the data via a light signal indicative of the data from the first communication partner device (47) to the second communication partner device (41).

2. The display arrangement of claim 1, wherein the first communication partner device is configured as a further head-up display (47) for visually displaying further data to the user, wherein the first mounting element is to be mounted at the first position of the diving apparatus so that the further head-up display (47) is in the field of view of the user.

3. The display arrangement of claim 1 or 2, comprising a mouthpiece (1) having a first mounting provision (21) at which the first mounting element of the first communication partner device (47) is mounted or mountable.

4. The display arrangement of any of claims 1 to 3, comprising a user mask (42, 45) having a second mounting provision (72) at which the second mounting element (43) of the head-up display (41) is mounted or mountable.

5. The display arrangement of any of claims 2 to 4, wherein the further head-up display (47) comprises at least one visible light source (56 for visually displaying the further data.

6. The display arrangement of any of claims 1 to 5, wherein the head-up display (41) comprises a two-dimensional display area (52) for visually displaying the data.

7. The display arrangement of any of claims 1 to 6, wherein the wireless communication link (49, 55) is configured for transmitting the light signal as infrared light or visible light.

8. The display arrangement of any of claims 1 to 7, wherein the wireless communication link (49, 55) is configured for transmitting a pulsed light signal.

9. The display arrangement of any of claims 1 to 8, wherein the wireless communication link (49, 55) comprises
a light emitting element (49) configured for emitting the light and being located at the first communication partner device (47); and
a light sensitive element (55) configured for sensing the emitted light and being located at the head-up display (41).

10. The display arrangement of any of claims 1 to 9, wherein the head-up display (41) comprises a plano-convex lens (48) having a planar surface being exposed to an environment of the display arrangement and having a convex surface being sealed against the environment.

11. The display arrangement of claim 10, wherein the head-up display (41) comprises a reflective member (50) arranged between the concave lens (79) and the plano-convex lens (48).

12. The display arrangement of any of claims 1 to 11, wherein the wireless communication link (49, 55) is a unidirectional communication link.

13. The display arrangement of any of claims 1 to 12, wherein at least one of the data and the further data is diving operation relevant data.

14. A rebreather for supplying a user with a breathing gas, wherein the rebreather comprises a display arrangement of any of claims 1 to 13.

15. The rebreather of claim 14, comprising one of the following features:
the rebreather is configured as a diving rebreather;
the rebreather is configured as a fire fighting rebreather.

## Patentansprüche

1. Anzeigeanordnung für eine Tauchvorrichtung, wobei die Anzeigeanordnung umfasst:
eine erste Kommunikationspartnervorrichtung (47) mit einem ersten Befestigungselement, das an einer ersten Position der Tauchvorrichtung anzubringen ist;
**dadurch gekennzeichnet, dass** die Anzeigeanordnung ferner eine zweite Kommunikationspartnervorrichtung umfasst, die als ein Head-up-Display (41) zum visuellen Anzeigen von Daten an einen Benutzer konfiguriert ist und ein zweites Befestigungselement (43) aufweist, das an einer zweiten Position der Tauchvorrichtung derart anzubringen ist, dass sich das Head-up-Display (41) im Sichtfeld des Benutzers befindet;
eine drahtlose Kommunikationsverbindung (49, 55),
die konfiguriert ist, die Daten über ein Lichtsignal indikativ für die Daten von der ersten Kommunikationspartnervorrichtung (47) zu der zweiten Kommunikationspartnervorrichtung (41) zu übertragen.

2. Anzeigeanordnung nach Anspruch 1, wobei die erste Kommunikationspartnervorrichtung als ein weiteres Head-up-Display (47) für visuelles Anzeigen weiterer Daten an einen Benutzer konfiguriert ist, wobei das erste Befestigungselement an der ersten Position der Tauchvorrichtung derart zu befestigen ist, dass das weitere Head-up-Display (47) in dem Sichtfeld des Benutzers liegt.

3. Anzeigeanordnung nach Anspruch 1 oder 2, umfassend ein Mundstück (1) mit einer ersten Befestigungsvorrichtung (21), an der das erste Befestigungselement der ersten Kommunikationspartnervorrichtung (47) befestigt oder befestigbar ist.

4. Anzeigeanordnung nach einem der Ansprüche 1 bis 3, umfassend eine Benutzermaske (42, 45) mit einer zweiten Befestigungsvorrichtung (72), an der das zweite Befestigungselement (43) des Head-up-Displays (41) befestigt oder befestigbar ist.

5. Anzeigeanordnung nach einem der Ansprüche 2 bis 4, wobei das weitere Head-up-Display (47) mindestens eine sichtbare Lichtquelle (56) zum visuellen Anzeigen der weiteren Daten umfasst.

6. Anzeigeanordnung nach einem der Ansprüche 1 bis 5, wobei das Head-up-Display (41) einen zweidimensionalen Anzeigebereich (52) zum visuellen Anzeigen der Daten umfasst.

7. Anzeigeanordnung nach einem der Ansprüche 1 bis 6, wobei die drahtlose Kommunikationsverbindung (49, 55) zum Übertragen des Lichtsignals als Infrarotlicht oder sichtbares Licht konfiguriert ist.

8. Anzeigeanordnung nach einem der Ansprüche 1 bis 7, wobei die drahtlose Kommunikationsverbindung (49, 55) für das Übertragen eines gepulsten Lichtsignals konfiguriert ist.

9. Anzeigeanordnung nach einem der Ansprüche 1 bis 8, wobei die drahtlose Kommunikationsverbindung (49, 55) umfasst
ein lichtemittierendes Element (49), das für das Emittieren des Lichts konfiguriert ist und sich an der ersten Kommunikationspartnervorrichtung (47) befindet; und
ein lichtempfindliches Element (55), das für das Erfassen des emittierten Lichts konfiguriert ist und sich an dem Head-up-Display (41) befindet.

10. Anzeigeanordnung nach einem der Ansprüche 1 bis 9, worin das Head-up-Display (41) eine plankonvexe Linse (48) mit einer planaren Fläche, die einer Umgebung der Anzeigeanordnung ausgesetzt ist, und mit einer konvexen Fläche, die gegen die Umgebung abgedichtet ist, umfasst.

11. Anzeigeanordnung nach Anspruch 10, wobei das Head-up-Display (41) ein reflektierendes Element (50) umfasst, das zwischen der konkaven Linse (79) und der plankonvexen Linse (48) angeordnet ist.

12. Anzeigeanordnung nach einem der Ansprüche 1 bis 11, wobei die drahtlose Kommunikationsverbindung (49, 55) eine unidirektionale Kommunikationsverbindung ist.

13. Anzeigeanordnung nach einem der Ansprüche 1 bis 12, wobei die Daten und/oder die weiteren Daten tauchbetriebsrelevante Daten sind.

14. Rückatmer zur Versorgung eines Benutzers mit einem Atemgas, wobei der Rückatmer eine Anzeigeanordnung nach einem der Ansprüche 1 bis 13 umfasst.

15. Rückatmer nach Anspruch 14, eines oder mehrere der folgenden Merkmale umfassend:
der Rückatmer ist als ein Tauchrückatmer konfiguriert;
der Rückatmer ist als ein Feuerbekämpfungsrückatmer konfiguriert.

## Revendications

1. Agencement d'affichage pour un appareil de plongée, l'agencement d'affichage comprenant :
un premier dispositif partenaire de communication (47) ayant un premier élément de montage destiné à être monté en une première position de l'appareil de plongée ;
**caractérisé en ce que** l'agencement d'affichage comprend en outre
un deuxième dispositif partenaire de communication configuré sous la forme d'un affichage tête haute (41) pour afficher visuellement des données pour un utilisateur et ayant un deuxième élément de montage (43) destiné à être monté en une deuxième position de l'appareil de plongée de sorte que l'affichage tête haute (41) soit dans le champ de vision de l'utilisateur ;
une liaison de communication sans fil (49, 55) configurée pour transmettre les données par l'intermédiaire d'un signal lumineux indiquant les données issues du premier dispositif partenaire de communication (47) au deuxième dispositif partenaire de communication (41).

2. Agencement d'affichage selon la revendication 1, dans lequel le premier dispositif partenaire de communication est configuré sous la forme d'un affichage tête haute complémentaire (47) pour afficher visuellement des données complémentaires pour l'utilisateur, dans lequel le premier élément de montage est destiné à être monté en la première position de l'appareil de plongée de sorte que l'affichage tête haute complémentaire (47) soit dans le champ de vision de l'utilisateur.

3. Agencement d'affichage selon la revendication 1 ou la revendication 2, comprenant un embout buccal (1) ayant une première disposition de montage (21) au niveau de laquelle le premier élément de montage du premier dispositif partenaire de communication (47) est monté ou montable.

4. Agencement d'affichage selon l'une quelconque des revendications 1 à 3, comprenant un masque d'utilisateur (42, 45) ayant une deuxième disposition de montage (72) au niveau de laquelle le deuxième élément de montage (43) de l'affichage tête haute (41) est monté ou montable.

5. Agencement d'affichage selon l'une quelconque des revendications 2 à 4, dans lequel l'affichage tête haute complémentaire (47) comprend au moins une source de lumière visible (56) pour afficher visuellement les données complémentaires.

6. Agencement d'affichage selon l'une quelconque des revendications 1 à 5, dans lequel l'affichage tête haute (41) comprend une zone d'affichage bidimensionnelle (52) pour afficher visuellement les données.

7. Agencement d'affichage selon l'une quelconque des revendications 1 à 6, dans lequel la liaison de communication sans fil (49, 55) est configurée pour transmettre le signal lumineux sous la forme de lumière infrarouge ou de lumière visible.

8. Agencement d'affichage selon l'une quelconque des revendications 1 à 7, dans lequel la liaison de communication sans fil (49, 55) est configurée pour transmettre un signal lumineux pulsé.

9. Agencement d'affichage selon l'une quelconque des revendications 1 à 8, dans lequel la liaison de communication sans fil (49, 55) comprend
un élément photoémetteur (49) configuré pour émettre la lumière et être situé au niveau du premier dispositif partenaire de communication (47) ; et
un élément photosensible (55) configuré pour détecter la lumière émise et être situé au niveau de l'affichage tête haute (41).

10. Agencement d'affichage selon l'une quelconque des revendications 1 à 9, dans lequel l'affichage tête haute (41) comprend une lentille piano-convexe (48) ayant une surface plane qui est exposée à un environnement de l'agencement d'affichage et ayant une surface convexe qui est hermétique à l'environnement.

11. Agencement d'affichage selon la revendication 10, dans lequel l'affichage tête haute (41) comprend un organe réfléchissant (50) agencé entre la lentille concave (79) et la lentille piano-convexe (48).

12. Agencement d'affichage selon l'une quelconque des revendications 1 à 11, dans lequel la liaison de communication sans fil (49, 55) est une liaison de communication unidirectionnelle.

13. Agencement d'affichage selon l'une quelconque des revendications 1 à 12, dans lequel au moins des données parmi les données et les données complémentaires sont des données relatives à une opération de plongée.

14. Appareil de respiration à circuit fermé pour fournir à un utilisateur un gaz respiratoire, l'appareil de respiration à circuit fermé comprenant un agencement d'affichage selon l'une quelconque des revendications 1 à 13.

15. Appareil de respiration à circuit fermé selon la revendication 14, comprenant l'une des caractéristiques suivantes :
l'appareil de respiration à circuit fermé est configuré sous la forme d'un appareil de respiration à circuit fermé de plongée ;
l'appareil de respiration à circuit fermé est configuré sous la forme d'un appareil de respiration à circuit fermé de lutte contre l'incendie.
